# EUROPÄISCHE PATENTSCHRIFT

(11) **EP 3 481 437 B1**
(45) Veröffentlichungstag und Bekanntmachung des Hinweises auf die Patenterteilung: **26.01.2022**
(21) Anmeldenummer: 17811189.4
(22) Anmeldetag: 17.11.2017
(51) Int. Cl.: A61K 51/04, A61K 101/02, C07D 213/82, C07B 59/00

(54) **PRÄKURSOREN FÜR DIE RADIOFLUORIERUNG**
PRECURSORS FOR RADIOFLUORINATION
PRÉCURSEURS DE RADIOFLUORATION

(30) Priorität: 18.11.2016 DE 102016122273
(43) Veröffentlichungstag der Anmeldung: 15.05.2019
(73) Patentinhaber: ABX Advanced Biochemical Compounds GmbH, 01454 Radeberg (DE)
(72) Erfinder: MARTIN, René, 01157 Dresden (DE); SMITS, René, 01097 Dresden (DE); HESSE, Ronny, 01069 Dresden (DE); HOEPPING, Alexander, 01324 Dresden (DE); MÜLLER, Marco, 01324 Dresden (DE); HÜBNER, Sandra, 01809 Heidenau (DE)
(74) Vertreter: Riechelmann & Carlsohn Patentanwälte PartG mbB
(86) Internationale Anmeldenummer: PCT/DE2017/100986
(87) Internationale Veröffentlichungsnummer: WO 2018/091043

(56) Entgegenhaltungen:
- WO-A1-2016/030329
- WO-A1-2017/027870
- WO-A1-2017/029399
- WO-A1-2017/054907
- WO-A1-2017/072200
- WO-A1-2017/117687
- WO-A1-2017/214470
- WO-A1-2018/005625
- WO-A1-2018/091043
- WO-A2-2010/014933
- WO-A2-2016/065142
- WO-A2-2016/065145
- WO-A2-2017/070482
- HAYDEN T. RAVERT ET AL: "An improved synthesis of the radiolabeled prostate-specific membrane antigen inhibitor, [ 18 F]DCFPyL : Radiolabeled PSMA inhibitor, [ 18 F]DCFPyL", JOURNAL OF LABELLED COMPOUNDS AND RADIOPHARMACEUTICALS, Bd. 59, Nr. 11, 1. September 2016 (2016-09-01), Seiten 439-450, XP055447890, CHICHESTER; GB ISSN: 0362-4803, DOI: 10.1002/jlcr.3430
- KELLY JAMES ET AL: "Synthesis and pre-clinical evaluation of a new class of high-affinity18F-labeled PSMA ligands for detection of prostate cancer by PET imaging", EUROPEAN JOURNAL OF NUCLEAR MEDICINE AND MOLECULAR IMAGING, SPRINGER VERLAG, HEIDELBERG, DE, Bd. 44, Nr. 4, 15. November 2016 (2016-11-15), Seiten 647-661, XP036183544, ISSN: 1619-7070, DOI: 10.1007/S00259-016-3556-5 [gefunden am 2016-11-15]
- Y. CHEN ET AL: "2-(3-?-Ureido)-Pentanedioic Acid, [18F]DCFPyL, a PSMA-Based PET Imaging Agent for Prostate Cancer", CLINICAL CANCER RESEARCH, Bd. 17, Nr. 24, 15. Dezember 2011 (2011-12-15), Seiten 7645-7653, XP055278472, US ISSN: 1078-0432, DOI: 10.1158/1078-0432.CCR-11-1357
- POETHKO THORSTEN ET AL: "Two-step methodology for high-yield routine radiohalogenation of peptides: 18F-labeled RGD and octreotide analogs", THE JOURNAL OF NUCLEAR MEDICINE, SOCIETY OF NUCLEAR MEDICINE, US, Bd. 45, Nr. 5, 1. Mai 2004 (2004-05-01), Seiten 892-902, XP002520745, ISSN: 0161-5505
- Jens Cardinale ET AL: "Preliminary Results on the Synthesis of 18F-PSMA-1007 by Direct One-Step Fluorination", Journal of Nuclear Medicine, MAY 1 2017, vol. 58, 10. Juni 2017 (2017-06-10), XP055447814, Annual Meeting of the Society-of-Nuclear-Medicine-and-Molecular- Imaging (SNMMI); Denver, CO, USA; June 10 -14, 2017 Gefunden im Internet: URL:http://jnm.snmjournals.org/content/58/ supplement_1/334.short?related-urls=yes&le gid=jnumed;58/supplement_1/334 [gefunden am 2018-02-05]
- POETHKO T ET AL: "Two-step methodology for high-yield routine radiohalogenation of peptides: 18F-labeled RGD and Octreotide Analogs", THE JOURNAL OF NUCLEAR MEDICINE, SOCIETY OF NUCLEAR MEDICINE, US, vol. 45, no. 5, 1 May 2004 (2004-05-01), pages 892-902, XP002383247, ISSN: 0161-5505
- Barbara Wenzel ET AL: "Development of a Novel Nonpeptidic 18 F-Labeled Radiotracer for in Vivo Imaging of Oxytocin Receptors with Positron Emission Tomography", Journal of Medicinal Chemistry, vol. 59, no. 5, 3 February 2016 (2016-02-03), pages 1800-1817, XP055447781, ISSN: 0022-2623, DOI: 10.1021/acs.jmedchem.5b01080
- Jan Tykvart ET AL: "Design of Highly Potent Urea-Based, Exosite-Binding Inhibitors Selective for Glutamate Carboxypeptidase II", Journal of Medicinal Chemistry, vol. 58, no. 10, 7 May 2015 (2015-05-07), pages 4357-4363, XP055448412, ISSN: 0022-2623, DOI: 10.1021/acs.jmedchem.5b00278
- Osama Sabri ET AL: "First-in-human PET quantification study of cerebral [alpha]4[beta]2* nicotinic acetylcholine receptors using the novel specific radioligand (-)-[ 18 F]Flubatine", NeuroImage, vol. 118, 1 September 2015 (2015-09-01), pages 199-208, XP055447787, AMSTERDAM, NL ISSN: 1053-8119, DOI: 10.1016/j.neuroimage.2015.05.065
- YING CHEN ET AL: "Radiohalogenated prostate-specific membrane antigen (PSMA)-based ureas as imaging agents for prostate cancer", JOURNAL OF MEDICINAL CHEMISTRY, AMERICAN CHEMICAL SOCIETY, vol. 51, 25 December 2008 (2008-12-25), pages 7933-7943, XP002614473, ISSN: 0022-2623, DOI: 10.1021/JM801055H [retrieved on 2008-12-03]
- MARK H. DORNAN ET AL: "Simplified and robust one-step radiosynthesis of [ 18 F]DCFPyL via direct radiofluorination and cartridge-based purification", JOURNAL OF LABELLED COMPOUNDS AND RADIOPHARMACEUTICALS, vol. 61, no. 10, 1 August 2018 (2018-08-01), pages 757-763, XP055516244, GB ISSN: 0362-4803, DOI: 10.1002/jlcr.3632

## Beschreibung

Die Erfindung betrifft Präkursoren für die Radiofluorierung sowie Verfahren zur Radiofluorierung der Präkursoren.

In der medizinischen Diagnostik werden kurzlebige, radioaktiv markierte Verbindungen, sogenannte Radiotracer, eingesetzt, deren physiologische und biochemische Eigenschaften eine nicht-invasive tomographische Erfassung von Stoffwechselprozessen im menschlichen Körper ermöglichen. Durch die Anwendung des modernen tomographischen Verfahrens der Positronen-Emissions-Tomographie (PET) können mithilfe dieser Radiotracer Stoffwechselprozesse quantifiziert und die Bioverteilung des Radiodiagnostikums von außen her erfasst werden. Die tomographische Erfassung von Radiotracern, wie z. B. 2-Deoxy-2-[¹⁸F]fluor-D-glucose ([¹⁸F]-FDG), ermöglicht eine frühzeitige Diagnose von Tumoren, die sich hinsichtlich des Glukosestoffwechsels von Normalgewebe signifikant unterscheiden. Durch die Entwicklung neuer Radiotracer auf der Basis pharmakologisch interessanter Verbindungen haben sich in den letzten Jahren neue Möglichkeiten der nicht invasiven Diagnostik verschiedener Krankheitsbilder eröffnet.

Der weltweite Anteil der Positronen-Emissions-Tomographie (PET) am Gesamtmarkt der Diagnose mittels bildgebender Verfahren ist in den letzten Jahren explosionsartig gestiegen. Den größten Anteil hierbei hat das [¹⁸F]Fluorid als radioaktive Sonde, da es in Form des F-18 markierten Zuckerderivates ([¹⁸F]-FDG) die genaue Lokalisation von Tumoren bis in den Millimeterbereich mittels PET sichtbar macht und eine genaue Lokalisation der Tumorausdehnung ermöglicht. Es hat sich allerdings herausgestellt, dass [¹⁸F]-FDG, welches häufig als das "Arbeitspferd" der Nuklearmedizin bezeichnet wird, zum Nachweis von primärem, organbegrenzten Prostatakrebs nur von beschränktem Nutzen ist (Bouvez et al., EJNMMI Research 2016, 6, 40). Aus diesem Grund sind für den Nachweis von Prostatatumoren und Metastasen, die verstärkt PSMA (Prostataspezifisches Membranantigen) exprimieren, neue Radiotracer wie [¹⁸F]-DCFPyL (Formel 1) und [¹⁸F]F-PSMA-1007 (Formel 2) entwickelt worden, die zum Nachweis des Prostataspezifischen Membranantigens (PSMA) eingesetzt werden können.

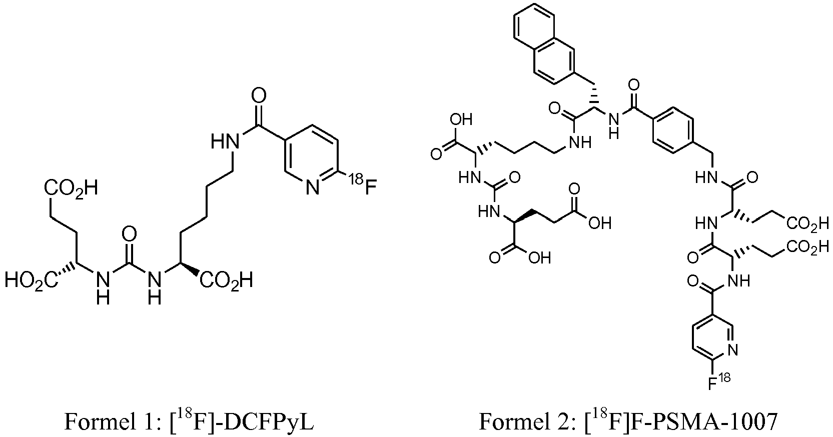

Es ist in den Formeln 1 und 2 zu erkennen, dass diese Radiotracer multifunktionale Moleküle sind, weil sie eine große Zahl an freien funktionellen Gruppen wie z. B. -OH, -CONH, -COOH aufweisen. Normalerweise sind Moleküle mit vielen freien funktionellen Gruppen nicht für die direkte Markierung mit ¹⁸F geeignet. Die funktionellen Gruppen reagieren oft mit dem [¹⁸F]-Fluorid-Anion, meist so, dass HF entsteht. Für eine erfolgreiche Radiomarkierung steht damit kein reaktives Fluorid mehr zur Verfügung. Weiterhin ist die Löslichkeit stark polarer Verbindungen in wasserfreien Lösungsmitteln stark herabgesetzt. In wässrigen Lösungsmitteln ist zudem das [¹⁸F]-Fluorid-Anion nicht stark genug aktiviert, daher bedient man sich in der Radiochemie sogenannter "nackter Anionen", bei denen in organischer Lösung das positive Zentrum des Gegenions wie z. B. bei Tetra-n-butyl-Ammonium-Salzen durch unpolare Kohlenwasserstoffketten abgeschirmt wird.

Stand der Technik ist daher bei diesen multifunktionalen Molekülen der Einbau von Schutzgruppen oder der Einbau über vorher radioaktiv markierte prosthetische Gruppen (beides zweistufige Reaktionen), so dass nur noch die synthetisch eingebrachte Abgangsgruppe (in diesem Fall Trimethylammonium-triflat) mit dem Tetra-*n*-butyl-Ammonium-Hydrogencarbonat-aktivierten [¹⁸F]-Fluorid-Anion reagieren kann. Diese Radiotracer werden üblicherweise aus Gründen des Strahlenschutzes in sogenannten "Heißen Zellen" mit automatisierten Synthesemodulen unter Verwendung von Einmalmaterialien wie z. B. Kassetten, insbesondere sterilisierten Kassetten, und Reagenzien hergestellt. Aufwendige, mehrstufige Syntheserouten lassen sich mit diesen Systemen oft nicht kosteneffizient realisieren.

Der Einbau von Schutzgruppen hat aber immer den Nachteil, dass man diese aufwendig durch Säuren oder Basen entschützen muss. Daher spricht man hier auch von 2-Stufen-Reaktionen: Erste Stufe: die Markierung mit ¹⁸F. Zweite Stufe: die Entschützung mit Säure oder Base. Sowohl der Einsatz von Schutzgruppen als auch der Einsatz von Säuren und Basen führt oftmals zu erheblichen Nebenprodukten, welche von der gewünschten ¹⁸F-markierten Substanz getrennt werden müssen. Dieses wird meistens apparativ aufwendig mittels einer HPLC (High-Performance-Liquid-Chromatographie) bewerkstelligt und ist dadurch zeit- und kostenintensiv. Die Synthese über prostethische Gruppen erfolgt ebenfalls über mindestens zwei Stufen. Zunächst wird die prostethische Gruppe mit ¹⁸F markiert, dann wird diese mit dem Zielmolekül gekoppelt.

Der Zeitfaktor spielt in der Radiopharmazie eine erhebliche Rolle, da das ¹⁸FluoridAnion eine Halbwertszeit von nur 109 Minuten hat und daher jede Art der Verlängerung von Synthesezeit und Transportweg direkte Auswirkungen auf die Menge der zu erzielenden Patientendosen zur Folge hat.

Zur Herstellung von 18F-DCFPyL sind demnach zwei Wege bekannt: Der erste Weg ist eine zweistufige Synthese mittels geschütztem Präkursor (Ravert et al., J. Label Compd. Radiopharm 2016, 59, 439-50; Schema 1, bzw. Bouvet et al., EJNMMI Research, 2016, 6: 40).

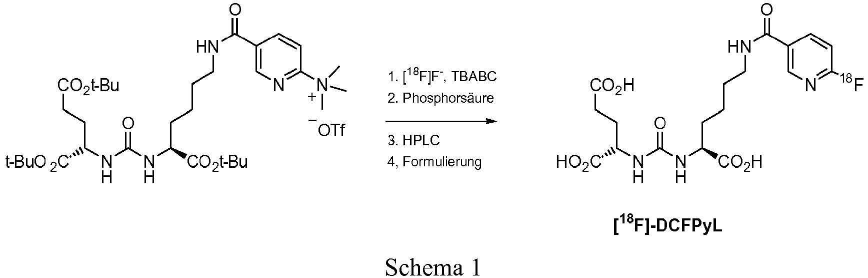

Die Carbonsäurefunktionalitäten im Präkursor sind als tert-Butylester geschützt. Das Endprodukt wird im zweiten Schritt mit Säure freigesetzt. Die Reinigung erfolgt über eine HPLC. Die Gesamtausbeute auf dem ELIXYS Mikrofluidikmodul beträgt im Mittel 19 % nach 87 min Synthesezeit.

Der zweite Weg ist eine dreistufige Synthese und verläuft über eine prosthetische Gruppe (Chen et al., Clin. Cancer Res. 2011, 17, 7645-53; Schema 2)

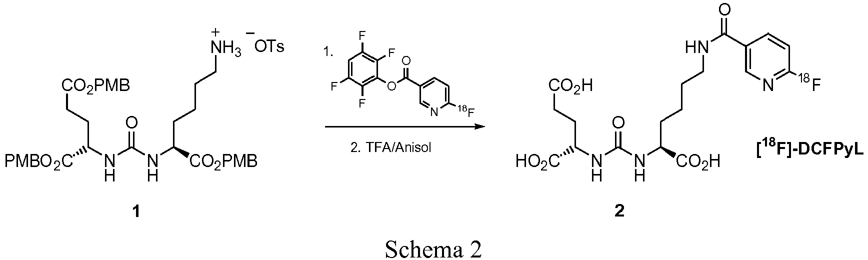

Dabei wird im ersten Schritt die prosthetische Gruppe 6-[¹⁸F]Fluomicotinsäure-2,3,5,6-tetrafluorphenylester hergestellt. Im zweiten Schritt wird das Ammoniumsalz (1) mit 6- [¹⁸F]Fluomicotinsäure-2,3,5,6-tetrafluorphenylester gekoppelt (a), und im dritten Schritt werden die PMB-Schutzgruppen mit TFA und Anisol entfernt (b). Man erhält dabei [¹⁸F]F-DCFPyL (2) in einer Gesamtausbeute von nur 5 bis 10 %.

Das in WO 2015/004029 A1 beschriebene Verfahren zur Radiofluorierung von Präkursoren erfordert die Überführung von Carboxylgruppen in Carboxylat-Anionen zur Ausbildung von Salzen mit kationischen Chelaten oder quaternären Ammoniumkationen. In einer der Radiofluorierung vorgeschalteten chemischen Synthese müssen die Präkursoren somit in Salze der Form K⁺/K222 überführt werden. Das Verfahren zielt insbesondere auf die Radiofluorierung freier Aminosäuren. Die Radiofluorierung wird im basischen Bereich durchgeführt. Die radiochemischen Ausbeuten übersteigen in keinem Fall 26 %.

Aus WO 2016/030 329 A1 ist ein mehrstufiges Verfahren zur Herstellung von [¹⁸F]-markierten Estern bekannt, die bei der Herstellung von PSMA-spezifischen PET-Tracern eingesetzt werden sollen.

Aufgabe der Erfindung ist es, die Nachteile nach dem Stand der Technik zu beseitigen. Es sollen insbesondere Präkursoren angegeben werden, die eine direkte Radiofluorierung mit hohen Ausbeuten an Radiotracern erlauben. Ferner soll ein Verfahren zur Radiofluorierung dieser Präkursoren angegeben werden.

Diese Aufgabe wird durch die Merkmale der Ansprüche 1, 9, 12, 13, 14 und 15 gelöst. Zweckmäßige Ausgestaltungen der Erfindungen ergeben sich aus den Merkmalen der Unteransprüche.

Nach Maßgabe der Erfindung ist ein Verfahren zur Herstellung einer radiofluorierten Verbindung, die einen aromatischen oder heteroaromatischen Ring, der [¹⁸F]-Fluor als ersten Substituenten trägt, eine Anbindungseinheit, die zur Anbindung an ein Peptid in der Lage ist, sowie eine Spacergruppe aufweist, die den aromatischen oder heteroaromatischen Ring mit der Anbindungseinheit verbindet. Der aromatische oder heteroaromatische Ring trägt [¹⁸F]-Fluor als ersten Substituenten. Die Anbindungseinheit trägt zumindest einen zweiten Substituenten, der aus der Gruppe ausgewählt ist, die aus -OH, -CONH und -COOH besteht. Die Anbindungseinheit ist mit der Spacergruppe über eine Bindung A¹ verbunden. Die Spacergruppe ist über eine Bindung A² mit dem aromatischen oder heteroaromatischen Ring verbunden. Das Verfahren umfasst die Schritte:
(a) Bereitstellen eines Präkursors, der den aromatischen oder heteroaromatischen Ring, der einen Substituenten Y trägt, die Anbindungseinheit, die zur Anbindung an das Peptid in der Lage ist und die zumindest einen zweiten Substituenten, der aus der Gruppe ausgewählt ist, die aus -OH, -CONH und -COOH besteht, trägt, sowie die Spacergruppe aufweist, wobei der aromatische oder heteroaromatische Ring, der einen Substituenten Y trägt, die allgemeine Formel VId 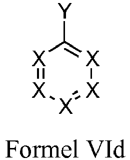 aufweist, wobei
   - X jeweils C-R⁸ oder N ist, mit der Maßgabe, dass höchstens zwei der Einheiten X N und der Rest der Einheiten X C-R⁸ sind und R⁸ unabhängig voneinander jeweils die Bindung A² zum Spacer, Wasserstoff oder unsubstituiertes oder substituiertes C₁-C₆-Alkyl sind, mit der Maßgabe, dass exakt ein Rest R⁸ eine Bindung A² zur Spacergruppe ist und die verbleibenden R⁸ gleich oder verschieden voneinander sind und jeweils Wasserstoff oder unsubstituiertes oder substituiertes C₁-C₆-Alkyl darstellen; und
   - der Substituent Y aus der Gruppe ausgewählt ist, die aus -N+(R¹R²R³),-NO₂, -Cl, -Br, -F oder -I besteht, und R¹, R² und R³ gleich oder verschieden voneinander sind und jeweils unsubstituiertes oder substituiertes C₁-C₆-Alkyl sind,
   wobei sich der Präkursor von der radiofluorierten Verbindung nur dadurch unterscheidet, dass der Substituent Y durch [¹⁸F]-Fluor ersetzt ist; und
(b) Umsetzen des Präkursors mit einem [¹⁸F]-Fluorid-Anion in Gegenwart eines Aktivierungssalzes zu der radiofluorierten Verbindung in einer einstufigen Synthese, wobei der Substituent Y durch [¹⁸F]-Fluorid ersetzt wird und wobei das Aktivierungssalz ein Kation mit der allgemeinen Formel N+(R⁴R⁵R⁶R⁷) aufweist, wobei R⁴, R⁵, R⁶ und R⁷ gleich oder verschieden voneinander sind und jeweils unsubstituiertes oder substituiertes C₁-C₆-Alkyl sind.

Erfindungsgemäß kann eine vollständige Automatisierung der Umsetzung des Präkursors mit [¹⁸F]-Fluorid-Anion in Gegenwart des Aktivierungssalzes zu der radiofluorierten Verbindung an einem herkömmlichen Synthesemodul mit anschließender Kartuschenaufreinigung vorgesehen sein. Dabei kann die radiofluorierte Verbindung in radiochemischen Ausbeuten von mehr als 40 % erhalten werden.

Der Ausdruck "C₁-C₆-Alkyl" bezieht sich auf geradkettige oder verzweigte gesättigte aliphatische Kohlenwasserstoffgruppen mit 1 bis 6 Kohlenstoffatomen. Beispiele von C₁-C₆-Alkyl-Gruppen sind Methyl, Ethyl, n-Propyl, iso-Propyl, n-Butyl, iso-Butyl, sec-Butyl, tert-Butyl, n-Pentyl, n-Hexyl.

Der Ausdruck "substituiertes C₁-C₆-Alkyl" bezieht sich auf C₁-C₆-Alkyl, wie oben definiert, das einen oder mehrere Substituenten aufweist, die aus der Gruppe ausgewählt sind, die aus NH₂, NH(C₁-C₄-alkyl), N(C₁-C₄-alkyl)₂, Halogen, C₁-C₄-Alkyl, OH, O(C₁-C₄-alkyl), NO₂, CN, CO₂H oder CO₂(C₁-C₄-Alkyl) besteht, wobei jede der vorstehenden C₁-C₄-Alkyl-Gruppen unsubstituiert oder mit zumindest einem Halogenatom substituiert ist. Der Ausdruck "Halogen" bezieht sich auf Fluor, Chlor, Brom und Iod.

In der vorliegenden Erfindung bezieht sich der Begriff Präkursor auf eine chemische Verbindung, die ohne den Einsatz von Schutzgruppen durch Radiofluorierung in eine radiochemische Verbindung, d. h. die radiofluorierte Verbindung, überführt werden kann. Der Präkursor weist keine Carboxylatgruppen, insbesondere keine Carboxylat-Anionen -COO⁻ auf. Der Präkursor weist somit auch keine Carboxylatgruppen in Form eines Salzes mit einem Kation, beispielweise einem kationischen Chelat oder einem quaternären Ammoniumkation auf.

Der Präkursor kann jedoch eine oder mehrere Carboxygruppen -COOH aufweisen. Mittels des erfindungsgemäßen Verfahrens ist es möglich, einen Präkursor, der eine oder mehrere Carboxygruppen aufweist, ohne Einsatz von Schutzgruppen in eine radiofluorierte Verbindung zu überführen. Der Präkursor unterscheidet sich von der radiofluorierten Verbindung lediglich dadurch, dass der Substituent Y durch [¹⁸F]-Fluor ersetzt ist. Der Substituent Y des Präkursors und der [¹⁸F]-Fluor-Substituent der radiofluorierten Verbindung befinden sich in der gleichen Position.

Der Substituent Y ist vorzugsweise -N+(R¹R²R³), wobei R¹, R² und R³ gleich oder verschieden voneinander sind und jeweils unsubstituiertes oder substituiertes C₁-C₆-Alkyl sind. Vorzugsweise sind R¹, R² und R³ gleich und Methyl oder Butyl, wobei Methyl besonders bevorzugt ist. In diesem Fall handelt es sich bei der Gruppe -N⁺(R¹R²R³), die der aromatische oder heteroaromatische Ring des Präkursors trägt, um eine quaternäre Trimethylammonium-Gruppe. Als Anion zu der Gruppe -N⁺(R¹R²R³) des Präkursors kann ein beliebiges Anion vorgesehen sein. Beispielsweise kann das Anion aus der Gruppe ausgewählt sein, die ein Fluor-, Iod-, Brom-, Chlor-, Sulfonat-, Sulfat-, Phosphat-, Alkylsulfat-, Arylsulfat- oder Carboxylat-Anion umfasst. Vorzugsweise kann als Anion ein Carboxylat-Anion vorgesehen sein, beispielsweise CF₃COO⁻, CH₃COO⁻, C₂H₅COO⁻ oder HCOO⁻.

Wenn R¹, R² und R³ in der Gruppe -N⁺(R¹R²R³) jeweils Methyl sind, so ist es bevorzugt, dass das Anion der Gruppe -N⁺(R¹R²R³) aus der Gruppe ausgewählt ist, die aus Trifluoracetat (CF₃COO⁻), Acetat (CH₃COO⁻), Propionat (C₂H₅COO⁻) oder Formiat (HCOO⁻) besteht.

Der aromatische oder heteroaromatische Ring des Präkursors und damit der radiochemischen Verbindung ist vorzugsweise ein monozyklischer Ring oder ein annelliertes Ringsystem mit zwei oder mehr Ringen, wobei ein monozyklischer Ring bevorzugt ist. Das Heteroatom eines heteroaromatischen Ringes ist vorzugsweise aus der Gruppe ausgewählt, die N, O und S umfasst. Vorzugsweise ist das Heteroatom N. Ein heteroaromatischer Ring kann ein oder mehrere Heteroatome aufweisen. Vorzugsweise weist der heteroaromatische Ring ein oder zwei Heteroatome, bevorzugt ein oder zwei Stickstoffatome auf. In diesem Fall kann der Ring ein Pyridin-Ring, ein Pyridazin-Ring, ein Pyrimidin-Ring oder ein Pyrazin-Ring sein.

Ein bevorzugter aromatischer oder heteroaromatischer Ring, der einen Substituenten Y trägt, ist in der allgemeinen Formel VI gezeigt: 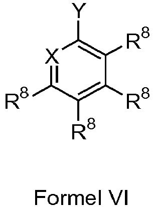 wobei X C-R⁸ oder N ist, Y -N⁺(R¹R²R³), -NO₂, -Cl, -Br, -F oder -I ist, wobei R¹, R² und R³ wie oben definiert sind und R⁸ unabhängig voneinander jeweils die Bindung A² zum Spacer, Wasserstoff oder unsubstituiertes oder substituiertes C₁-C₆-Alkyl sind, mit der Maßgabe, dass exakt ein Rest R⁸ eine Bindung A² zur Spacergruppe ist und die verbleibenden R⁸ gleich oder verschieden voneinander sind und jeweils Wasserstoff oder unsubstituiertes oder substituiertes C₁-C₆-Alkyl darstellen.

Ein besonders bevorzugter aromatischer oder heteroaromatischer Ring weist als Substituent Y die Gruppe -N⁺(R¹R²R³) und die Bindung A² in para-Stellung zur Gruppe -N⁺(R¹R²R³) auf, wie in der allgemeinen Formel VIa gezeigt ist: 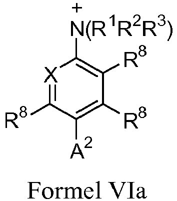 wobei X C-R⁸ oder N ist, R¹, R² und R³ wie oben definiert sind und R⁸ unabhängig voneinander jeweils Wasserstoff oder unsubstituiertes oder substituiertes C₁-C₆-Alkyl sind.

Ein stärker bevorzugter aromatischer oder heteroaromatischer Ring, der eine Gruppe -N⁺(R¹R²R³) als Substituent Y trägt, weist die Bindung A² in para-Stellung zur Gruppe -N⁺(R¹R²R³) auf, wie in der allgemeinen Formel VIb gezeigt ist: 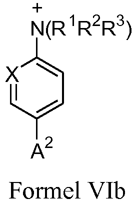 wobei X C-R⁸ oder N ist, R¹, R² und R³ wie oben definiert sind und R⁸ Wasserstoff oder unsubstituiertes oder substituiertes C₁-C₆-Alkyl ist. Bevorzugt sind X N und R¹, R² und R³ wie oben definiert.

Eine besonders bevorzugte Ausführungsform des aromatischen oder heteroaromatischen Ringes, der eine Gruppe -N⁺(R¹R²R³) als Substituent Y trägt, ist in Formel VIc gezeigt: 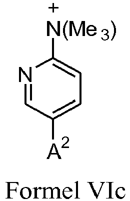 wobei Me für Methyl steht. In Formel VIc sind, bezogen auf die allgemeine Formel VI, X gleich N; R¹, R² und R³ Methyl; R⁸ in para-Stellung zur -N⁺(R¹R²R³)-Gruppe A² und die übrigen R⁸ Wasserstoff.

Der aromatische oder heteroaromatische Ring, der einen Substituenten Y trägt, ist in der allgemeinen Formel VId gezeigt: 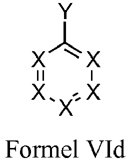 wobei
- X jeweils C-R⁸ oder N ist, mit der Maßgabe, dass höchstens zwei der Einheiten X N und der Rest der Einheiten X C-R⁸ sind; und
- Y -N+(R¹R²R³), -NO₂, -Cl, -Br, -F oder -I ist, wobei R¹, R² und R³ wie oben definiert sind und R⁸ unabhängig voneinander jeweils die Bindung A² zum Spacer, Wasserstoff oder unsubstituiertes oder substituiertes C₁-C₆-Alkyl sind, mit der Maßgabe, dass exakt ein Rest R⁸ eine Bindung A² zur Spacergruppe ist und die verbleibenden R⁸ gleich oder verschieden voneinander sind und jeweils Wasserstoff oder unsubstituiertes oder substituiertes C₁-C₆-Alkyl darstellen.

Die Anbindungseinheit ist zur Anbindung an ein Peptid in der Lage. Über die Anbindungseinheit kann die radiochemische Verbindung beispielsweise an eine funktionelle Einheit des Peptids spezifisch ankoppeln. Die Anbindungseinheit des Präkursors und damit der radiochemischen Verbindung kann eine Anbindungseinheit der allgemeinen Formel I sein: 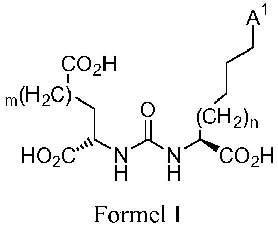 wobei A¹ die Bindung ist, über die die Anbindungseinheit mit der Spacergruppe verbunden ist, und m und n gleich oder verschieden voneinander sind und jeweils eine Ganzzahl von 0 bis 10 sind. Damit können m und n unabhängig voneinander jeweils 0, 1, 2, 3, 4, 5, 6, 7, 8, 9 oder 10 sein. Vorzugsweise ist m = 1 und n = 1. Die in Formel I gezeigte Anbindungseinheit ist zur Bindung an das Protein PSMA (prostataspezifisches Membranantigen) in der Lage. PSMA ist ein Protein, das in der Prostata eines Säugers exprimiert wird. Es wird bei Prostatakrebs, im Vergleich zu einer gesunden Prostata, in höherem Maße exprimiert.

Die Spacergruppe des Präkursors und damit der radiochemischen Verbindung ist vorzugsweise eine Spacergruppe der allgemeinen Formel II oder der allgemeinen Formel III: 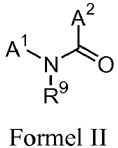 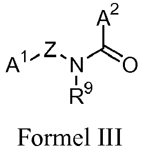 wobei A¹ die Bindung ist, über die die Spacergruppe mit der Anbindungseinheit verbunden ist, A² die Bindung ist, über die die Spacergruppe mit dem aromatischen oder heteroaromatischen Ring des Präkursors oder der radiochemischen Verbindung verbunden ist, R⁹ Wasserstoff oder eine unsubstituierte oder substituierte C₁-C₆-Alkylgruppe ist, und Z ein unsubstituierter oder ein- oder mehrfach substituierter Kohlenwasserstoff ist. Vorzugsweise ist R⁹ Wasserstoff.

In einer Ausführungsform der Erfindung ist Z eine Gruppe der Formel VII:

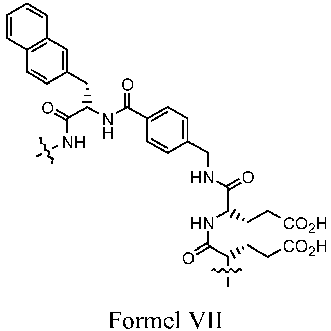

Eine Spacergruppe der Formel III, deren Gruppe Z die in Formel VII gezeigte Bedeutung hat, ist in Formel IIIa gezeigt: 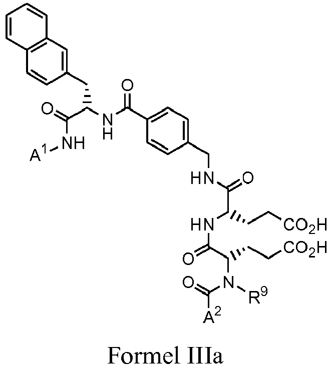 wobei R⁹ wie oben definiert ist. Vorzugsweise ist R⁹ Wasserstoff.

Erfindungsgemäß ist das Umsetzen des Präkursors mit einem [¹⁸F]-Fluorid-Anion in Gegenwart eines Aktivierungssalzes zu der radiofluorierten Verbindung vorgesehen. Dabei wird der Substituent Y durch [¹⁸F]-Fluorid ersetzt. Das Aktivierungssalz dient zur Aktivierung des [¹⁸F]-Fluorid-Anions. Das Aktivierungssalz weist ein Kation mit der allgemeinen Formel N+(R⁴R⁵R⁶R⁷) auf, wobei R⁴, R⁵, R⁶ und R⁷ gleich oder verschieden voneinander sind und jeweils unsubstituiertes oder substituiertes C₁-C₆-Alkyl sind. Es hat sich herausgestellt, dass solche Aktivierungssalze nicht nur für die Umsetzung der hier beschriebenen Präkursoren zu radiofluorierten Verbindungen, sondern auch für die Radiofluorierung anderer Präkursoren geeignet sind. Vorzugsweise sind R⁴, R⁵, R⁶ und R⁷ jeweils unsubstituiertes C₁-C₆-Alkyl, stärker bevorzugt Propyl, Butyl oder Pentyl, besonders bevorzugt jeweils n-Butyl. Vorzugsweise weist das Aktivierungssalz ein Anion auf, das aus der Gruppe ausgewählt ist, die Hydrogencarbonat (HCO₃⁻), Hydrogensulfat (HSO₄⁻), Oxalat, Phosphat und Toluensulfonat umfasst. Hydrogencarbonat und Phosphat sind stark bevorzugt. Phosphat ist besonders bevorzugt. In dem Aktivierungssalz liegen das Kation mit der allgemeinen Formel N+(R⁴R⁵R⁶R⁷) und das Anion in einem stöchiometrischen Verhältnis vor. In einer bevorzugten Ausführungsform ist das Aktivierungssalz Tetra-n-Butyl-Ammonium-Hydrogencarbonat oder Tetra-n-Butyl-Ammonium-phosphat, wobei Tetra-n-Butyl-Ammonium-phosphat besonders bevorzugt ist. Ein Aktivierungssalz, das das Kation Tetra-*n*-Butyl-Ammonium aufweist, wird im Folgenden auch als TBA bezeichnet.

Der Einsatz eines Aktivierungssalzes, das ein Kation mit der allgemeinen Formel N⁺(R⁴R⁵R⁶R⁷), wobei R⁴, R⁵, R⁶ und R⁷ gleich oder verschieden voneinander sind und jeweils unsubstituiertes oder substituiertes C₁-C₆-Alkyl sind, und Hydrogensulfat, Oxalat, Phosphat und Toluensulfonat als Anion aufweist, zur Aktivierung von [¹⁸F]-Fluorid-Anionen ist bisher unbekannt. Diese Aktivierungssalze sind nicht auf die Umsetzung der hier beschriebenen Präkursoren zu radiofluorierten Verbindungen beschränkt, sondern können auch für die Radiofluorierung anderer Präkursoren verwendet werden. Vorzugsweise sind R⁴, R⁵, R⁶ und R⁷ in dem Hydrogensulfat-, Oxalat-, Phosphat- und Toluensulfonat-Salz jeweils unsubstituiertes C₁-C₆-Alkyl, stärker bevorzugt Propyl, Butyl oder Pentyl, besonders bevorzugt jeweils n-Butyl. Hydrogencarbonat- und Phosphat-Salze sind stark bevorzugt. Phosphat-Salz ist besonders bevorzugt. In einer bevorzugten Ausführungsform ist das Aktivierungssalz Tetra-n-Butyl-Ammonium-Hydrogencarbonat oder Tetra-n-Butyl-Ammonium-phosphat, wobei Tetra-n-Butyl-Ammonium-phosphat besonders bevorzugt ist.

Das Aktivierungssalz liegt vorzugsweise in einer polaren Lösung, besonders bevorzugt in einer Lösung mit Wasser oder einem wasserhaltigen Lösungsmittel-Gemisch vor. Bei dem Lösungsmittel-Gemisch kann es sich beispielsweise um mit einem Alkohol wie Ethanol versetztes Wasser handeln. Der Alkoholzusatz dient zur Stabilisierung der Lösung. Das Aktivierungssalz kann beispielsweise als 0,001 bis 0,1 M Lösung, insbesondere als 0,075 M Lösung bereitgestellt werden.

Bei der radiofluorierten Verbindung handelt es sich beispielsweise um [¹⁸F]-DCFPyL (siehe Formel 1, oben) oder um [¹⁸F]F-PSMA-1007 (siehe Formel 2, oben). Ein zur Herstellung von [¹⁸F]-DCFPyL bevorzugter Präkursor ist eine Verbindung der allgemeinen Formel IV: 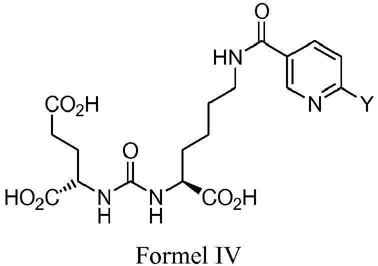 wobei der Substituent Y aus der Gruppe ausgewählt ist, die aus -N+(R¹R²R³), -NO₂, -Cl, -Br, -F oder -I besteht, und R¹, R² und R³ gleich oder verschieden voneinander sind und jeweils unsubstituiertes oder substituiertes C₁-C₆-Alkyl sind. Die Anbindungseinheit des in Formel IV gezeigten Präkursors entspricht der in Formel I gezeigten Anbindungseinheit, wobei m und n jeweils 1 sind.

Ein zur Herstellung von [¹⁸F]-DCFPyL stärker bevorzugter Präkursor ist eine Verbindung der allgemeinen Formel IVa: 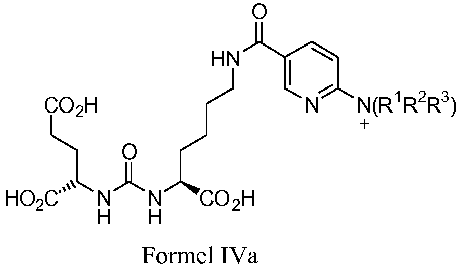 wobei R¹, R² und R³ gleich oder verschieden voneinander sind und jeweils unsubstituiertes oder substituiertes C₁-C₆-Alkyl sind. Besonders bevorzugt sind R¹, R² und R³ jeweils Methyl. Ein Präkursor der Formel IVa, bei dem R¹, R² und R³ jeweils Methyl sind, ist in Formel IVb veranschaulicht.

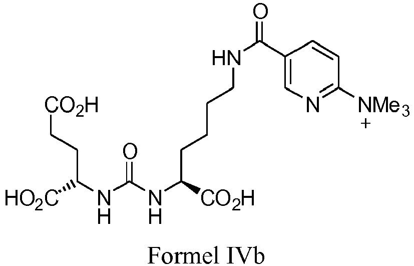

Ein zur Herstellung von [¹⁸F]F-PSMA-1007 bevorzugter Präkursor ist eine Verbindung der allgemeinen Formel V: 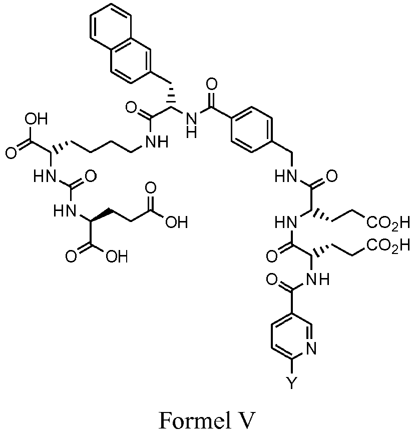 wobei der Substituent Y aus der Gruppe ausgewählt ist, die aus -N+(R¹R²R³), -NO₂, - Cl, -Br, -F oder -I besteht, und R¹, R² und R³ gleich oder verschieden voneinander sind und jeweils unsubstituiertes oder substituiertes C₁-C₆-Alkyl sind. Die Anbindungseinheit des in Formel V gezeigten Präkursors entspricht der in Formel I gezeigten Anbindungseinheit, wobei m und n jeweils 1 sind.

Ein zur Herstellung von [¹⁸F]F-PSMA-1007 stärker bevorzugter Präkursor ist eine Verbindung der allgemeinen Formel Va: 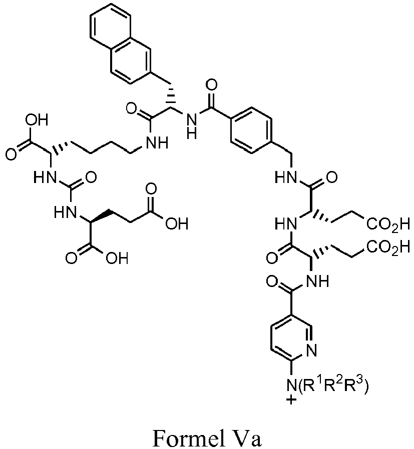 wobei R¹, R² und R³ gleich oder verschieden voneinander sind und jeweils unsubstituiertes oder substituiertes C₁-C₆-Alkyl sind. Besonders bevorzugt sind R¹, R² und R³ jeweils Methyl. Ein Präkursor der Formel Va, bei dem R¹, R² und R³ jeweils Methyl sind, ist in Formel Vb veranschaulicht.

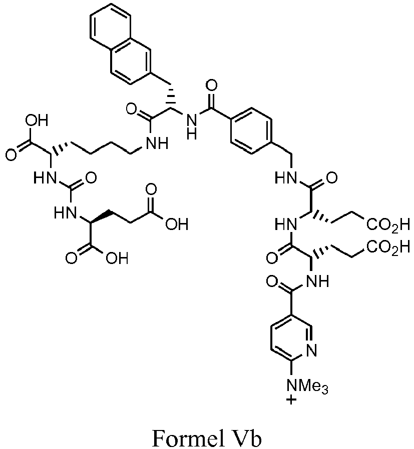

Der Präkursor wird vorzugsweise in einem nicht-protischen, polaren Lösungsmittel wie Acetonitril, Dimethylformamid (DMF), N,N-Dimethylacetamid (DMAA), N-Methyl-2-pyrrolidon (NMP), Dimethylsulfoxid (DMSO) oder Gemischen davon bereitgestellt.

Das in Schritt (b) eingesetzte [¹⁸F]-Fluorid-Anion kann mittels bekannter Verfahren hergestellt werden. Beispielsweise wird das [¹⁸F]-Fluorid-Anion im Zyklotron durch Bestrahlung von mindestens 97%-ig angereichertem H₂¹⁸O mit Protonen einer Energie von 9,6 MeV hergestellt. Die auf diesem Wege erhaltene wässrige [¹⁸F]-Fluoridlösung wird auf einer Anionentauscherkartusche (QMA) fixiert und mit Hilfe eines Phasen-Transfer-Katalysators (PTC), wie Kronenether, quartäre Ammoniumsalze oder Alkali- oder Erdalkalisalze, in ein Reaktionsgefäß überführt. Als PTC werden vorzugsweise ein [2,2,2]-Kryptand (Kryptofix^{®} oder K222), Tetra-n-Butyl-Ammonium-phosphat, -hydroxid, -oxalat, Toluensulfonat, oder wahlweise andere Kronenether wie 18-Krone-6 genutzt. Nach einer azeotropen Trocknung wird der Präkursor in einem organischen Lösungsmittel gelöst und zur getrockneten Reaktionsmischung dazugegeben. Bei dem organischen Lösungsmittel kann es sich um ein nichtprotisches, polares Lösungsmittel wie Acetonitril, Dimethylformamid (DMF), N,N-Dimethylacetamid (DMAA), N-Methyl-2-pyrrolidon (NMP), Dimethylsulfoxid (DMSO) oder Gemische davon handeln. Vorzugsweise wird Dimethylsulfoxid als Lösungsmittel verwendet.

Schritt (b) wird vorzugsweise unter thermischer Reaktionsführung im geschlossenen Reaktionsgefäß bei erhöhter Temperatur durchgeführt. Schritt (b) des erfindungsgemäßen Verfahrens wird vorzugsweise in einem nicht-protischen, polaren Lösungsmittel wie Acetonitril, Dimethylformamid (DMF), Dimethylsulfoxid (DMSO) oder Gemische davon durchgeführt. Vorzugsweise wird Dimethylsulfoxid als Lösungsmittel verwendet. Vorzugsweise wird das Verfahren bei einem pH-Wert von 1 bis 8 durchgeführt. Bevorzugt liegt der pH-Wert in einem Bereich von 4 bis 8, besonders bevorzugt bei 5. Das Verfahren kann aber auch bei einem pH-Wert größer 8 durchgeführt werden, wobei jedoch niedrigere Ausbeuten erzielt werden. Die Erfinder haben überraschenderweise gefunden, dass gerade bei einem pH-Wert im Bereich von 4 bis 8 weniger Nebenverbindungen entstehen und eine extrem hohe Markierungsausbeute erzielt werden kann.

Schritt (b) des erfindungsgemäßen Verfahrens wird bevorzugt für einen Zeitraum von 1 bis 60 min, stärker bevorzugt 3 bis 30 min und besonders bevorzugt 8 bis 20 min durchgeführt.

Schritt (b) des erfindungsgemäßen Verfahrens wird bevorzugt bei Temperaturen unter 100 °C, stärker bevorzugt bei Temperaturen zwischen Raumtemperatur und 95 °C, noch stärker bevorzugt zwischen Raumtemperatur und 90 °C und besonders bevorzugt bei Temperaturen zwischen 70 und 90 °C durchgeführt.

Schritt (b) des erfindungsgemäßen Verfahrens kann auch als mikrowellengestützte Reaktion durchgeführt werden. Dazu werden auf ein geschlossenes spezielles Reaktionsgefäß Mikrowellen mit einer Leistung von 50 bis 150 W, bevorzugt 75 bis 85 W eingestrahlt.

Zur Bestimmung der Markierungsausbeute und radioaktiven Nebenprodukte kann die Dünnschichtchromatographie (DC) und die High Performance Liquid Chromatography (HPLC) herangezogen werden.

Schema 3 veranschaulicht eine bevorzugte Ausführungsform des erfindungsgemäßen Verfahrens. Dabei wird ein Präkursor der Formel IV zu [¹⁸F]-DCFPyL in Gegenwart von [¹⁸F]-Fluorid-Anionen und einem Aktivierungssalz umgesetzt:

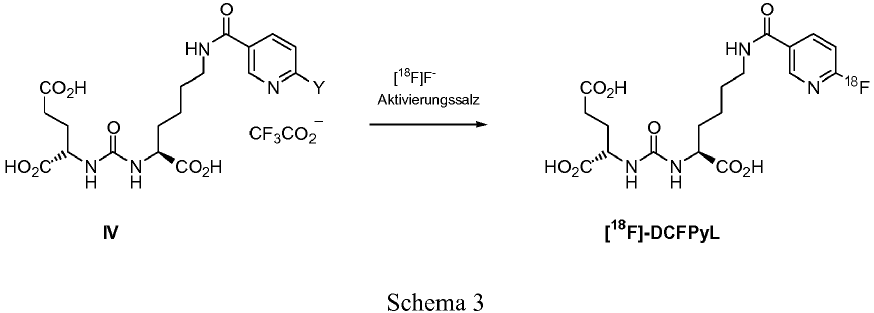

Es sei darauf hingewiesen, dass der Präkursor anstelle des Anions CF₃COO⁻ ein anderes Anion aufweisen kann. Vorzugsweise ist das Aktivierungssalz TBA, besonders bevorzugt TBA-Phosphat.

Schema 3a veranschaulicht eine bevorzugte Ausführungsform des erfindungsgemäßen Verfahrens. Dabei wird ein Präkursor der Formel IVa zu [¹⁸F]-DCFPyL in Gegenwart von [¹⁸F]-Fluorid-Anionen und einem Aktivierungssalz umgesetzt:

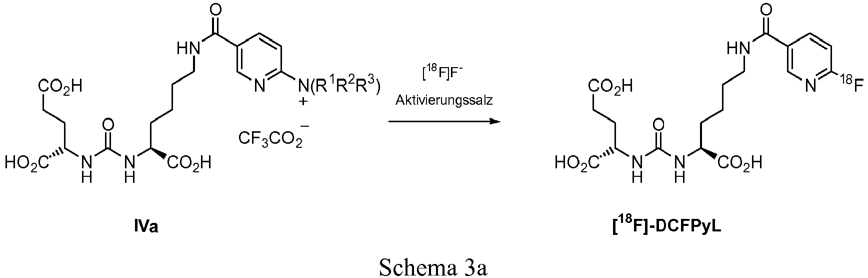

Es sei darauf hingewiesen, dass der Präkursor anstelle des Anions CF₃COO⁻ ein anderes Anion aufweisen kann. Vorzugsweise ist das Aktivierungssalz TBA, besonders bevorzugt TBA-Phosphat.

In einer bevorzugten Ausführungsform wird ein Präkursor der Formel IVb zur Herstellung von [¹⁸F]-DCFPyL in Gegenwart von [¹⁸F]-Fluor-Anionen und TBA, vorzugsweise TBA-Phosphat, als Aktivierungssalz umgesetzt, wie in Schema 3b gezeigt ist.

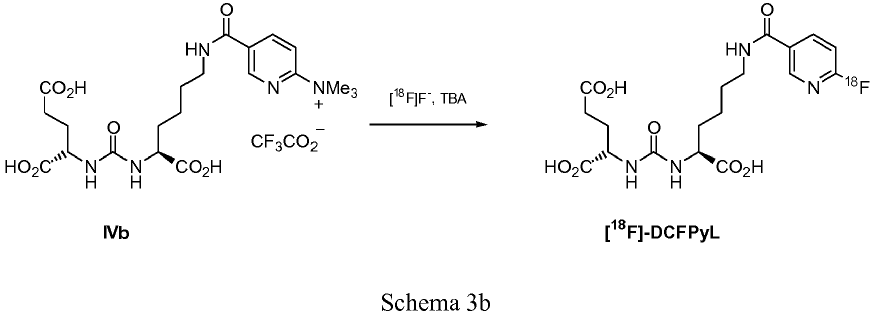

Es sei darauf hingewiesen, dass der Präkursor anstelle des Anions CF₃COO⁻ ein anderes Anion aufweisen kann.

Schema 4 veranschaulicht eine bevorzugte Ausführungsform des erfindungsgemäßen Verfahrens. Dabei wird ein Präkursor der Formel V zu [¹⁸F]F-PSMA-1007 in Gegenwart von [¹⁸F]-Fluorid-Anionen und einem Aktivierungssalz umgesetzt:

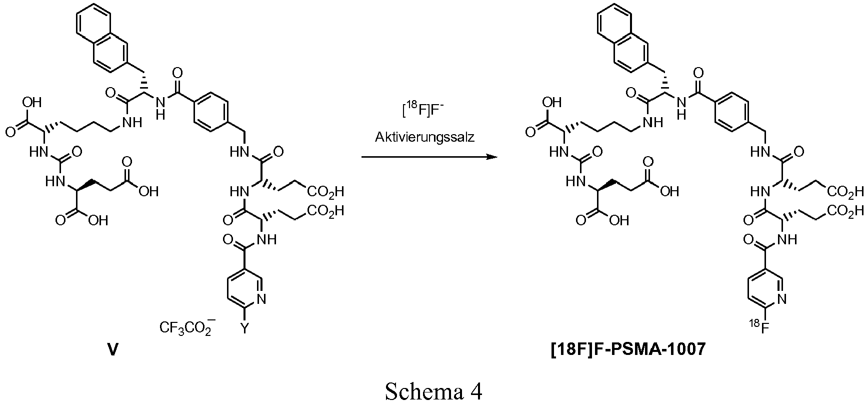

Es sei darauf hingewiesen, dass der Präkursor anstelle des Anions CF₃COO⁻ ein anderes Anion aufweisen kann. Vorzugsweise ist das Aktivierungssalz TBA, besonders bevorzugt TBA-Phosphat.

Schema 4a veranschaulicht eine bevorzugte Ausführungsform des erfindungsgemäßen Verfahrens. Dabei wird ein Präkursor der Formel Va zu [¹⁸F]F-PSMA-1007 in Gegenwart von [¹⁸F]-Fluorid-Anionen und einem Aktivierungssalz umgesetzt:

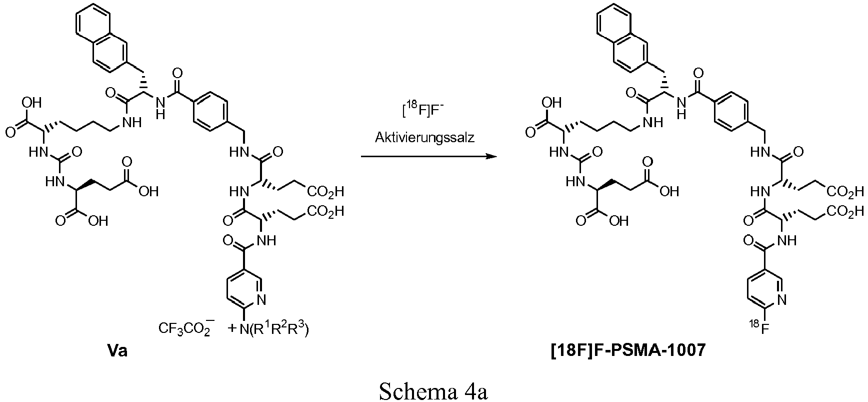

Es sei darauf hingewiesen, dass der Präkursor anstelle des Anions CF₃COO⁻ ein anderes Anion aufweisen kann. Vorzugsweise ist das Aktivierungssalz TBA, besonders bevorzugt TBA-Phosphat.

In einer besonders bevorzugten Ausführungsform wird ein Präkursor der Formel Vb zur Herstellung von [¹⁸F]F-PSMA-1007 in Gegenwart von [¹⁸F]-Fluor-Anionen und TBA, vorzugsweise TBA-Phosphat, als Aktivierungssalz umgesetzt, wie in Schema 4b gezeigt ist.

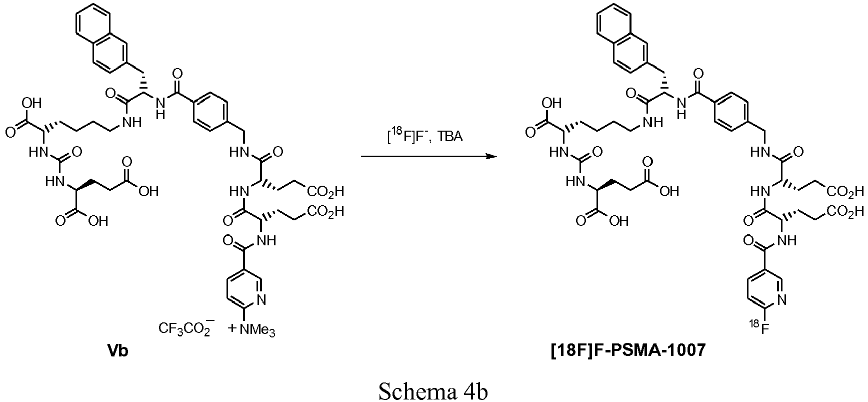

Es sei darauf hingewiesen, dass der Präkursor anstelle des Anions CF₃COO⁻ ein anderes Anion aufweisen kann.

Nach Maßgabe der Erfindung ist ferner die Verwendung eines Präkursors für die Herstellung einer radiofluorierten Verbindung in einer einstufigen Synthese vorgesehen. Der Präkursor weist einen aromatischen oder heteroaromatischen Ring, der einen Substituenten Y trägt, eine Anbindungseinheit, die zur Anbindung an ein Peptid in der Lage ist, sowie eine Spacergruppe auf, die den aromatischen oder heteroaromatischen Ring mit der Anbindungseinheit verbindet. Die Anbindungseinheit trägt zumindest einen zweiten Substituenten, der aus der Gruppe ausgewählt ist, die aus -OH, -CONH und -COOH besteht, wobei die Anbindungseinheit mit der Spacergruppe über eine Bindung A¹ und die Spacergruppe über eine Bindung A² mit dem aromatischen oder heteroaromatischen Ring verbunden ist, wobei der aromatische oder heteroaromatische Ring, der einen Substituenten Y trägt, die allgemeine Formel VId 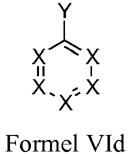 aufweist, wobei
- X jeweils C-R⁸ oder N ist, mit der Maßgabe, dass höchstens zwei der Einheiten X N und der Rest der Einheiten X C-R⁸ sind und R⁸ unabhängig voneinander jeweils die Bindung A² zum Spacer, Wasserstoff oder unsubstituiertes oder substituiertes C₁-C₆-Alkyl sind, mit der Maßgabe, dass exakt ein Rest R⁸ eine Bindung A² zur Spacergruppe ist und die verbleibenden R⁸ gleich oder verschieden voneinander sind und jeweils Wasserstoff oder unsubstituiertes oder substituiertes C₁-C₆-Alkyl darstellen; und
- der Substituent Y aus der Gruppe ausgewählt ist, die aus -N+(R¹R²R³), -NO₂, - Cl, -Br, -F oder -I besteht, und R¹, R² und R³ gleich oder verschieden voneinander sind und jeweils unsubstituiertes oder substituiertes C₁-C₆-Alkyl sind,
wobei sich der Präkursor von der radiofluorierten Verbindung nur dadurch unterscheidet, dass der Substituent Y durch [¹⁸F]-Fluor ersetzt ist. Weitere Einzelheiten des erfindungsgemäßen Präkursors sind oben im Zusammenhang mit dem erfindungsgemäßen Verfahren beschrieben worden.

Nach Maßgabe der Erfindung ist außerdem die Verwendung des erfindungsgemäßen Präkursors zur Herstellung einer radiofluorierten Verbindung, die einen aromatischen oder heteroaromatischen Ring, der [¹⁸F]-Fluor als ersten Substituenten trägt, eine Anbindungseinheit, die zur Anbindung an ein Peptid in der Lage ist, sowie eine Spacergruppe aufweist, die den aromatischen oder heteroaromatischen Ring mit der Anbindungseinheit verbindet, vorgesehen. Der aromatische oder heteroaromatische Ring trägt [¹⁸F]-Fluor als ersten Substituenten. Die Anbindungseinheit kann zumindest einen zweiten Substituenten tragen, der aus der Gruppe ausgewählt ist, die aus -OH, -CONH und -COOH besteht, wobei die Anbindungseinheit mit der Spacergruppe über eine Bindung A¹ und die Spacergruppe über eine Bindung A² mit dem aromatischen oder heteroaromatischen Ring verbunden ist.

Die Erfindung ermöglicht eine einstufige Synthese der aufgezeigten radiofluorierten Verbindungen. Damit wird zum einen eine Verkürzung der Synthesezeit erreicht. Zum anderen können die erzielten Markierungsausbeuten gegenüber dem in der Literatur bekannten zweistufigen Prozess mehr als doppelt so hoch liegen. Zudem lässt sich das Reaktionsprodukt einer einstufigen Synthese leichter aufreinigen, womit auf eine apparativ aufwendige HPLC verzichtet werden kann. Die radiofluorierten Verbindungen können sehr leicht und weniger zeitintensiv mit Kartuschen, sogenannten SPE-Kartuschen, aufgereinigt werden. Ferner ist die Vermeidung von konzentrierten Säuren und Basen im GMP-Umfeld (GMP = Good Manufacturing Practice) zu bevorzugen, da häufig korrosionsanfälliger Edelstahl in GMP-Bereichen verbaut ist. Die Einfachheit des erfindungsgemäßen Verfahrens erlaubt eine automatisierte Synthese der erfindungsgemäßen radiofluorierten Verbindung, beispielsweise mittels Einmal-Kassette und Reagenzienkit auf einem gängigen Synthesemodul. Die Reinigung kann während der Synthese mit Hilfe der SPE-Kartuschen erfolgen, so dass nach Ende der Synthese eine applikationsfertige Lösung der erfindungsgemäßen radiofluorierten Verbindung bereitgestellt werden kann.

Der erfindungsgemäße Präkursor trägt den zumindest einen zweiten Substituenten, d. h. zumindest eine ungeschützte OH-, CONH- und/oder COOH-Gruppe, den auch die Zielverbindung, d. h. die radiofluorierte Verbindung trägt. Es war nicht zu erwarten, dass Radiomarkierungen an Verbindungen, die ungeschützte OH-, CONH- und COOH-Gruppen tragen, funktionieren. Mit der vorliegenden Erfindung kann in der Praxis eine wesentliche Vereinfachung der automatisierten Synthese und damit der Herstellung der radiofluorierten Verbindung erreicht werden, da die Synthese nicht mehr in wie bisher mindestens zwei, sondern nun in einer Stufe unter einer deutlichen Zeitersparnis möglich ist. Die Zeitersparnis erlaubt eine wesentlich gesteigerte Ausbeute und damit eine einfachere und höhere Verfügbarkeit der radiofluorierten Verbindung. Damit kann aus einer Radiosynthese mehr Aktivität gewonnen werden, und somit können, wenn die bei der Radiosynthese hergestellte radiofluorierte Verbindung als Radiotracer verwendet wird, mehr Patienten untersucht werden.

Die Erfindung wird nachstehend anhand von Ausführungsbeispielen, näher erläutert.

### Beispiel 1

### Synthese eines Präkursors der Formel IVb

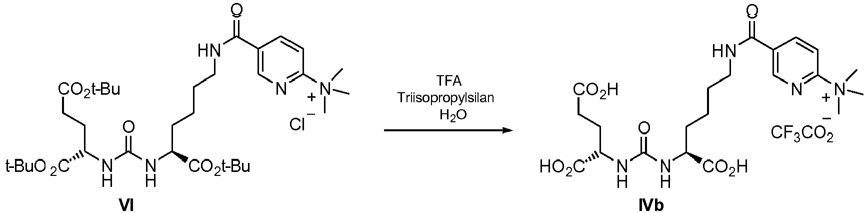

Die Synthese der Ausgangsverbindung VI erfolgte analog wie in der Literatur beschrieben (Ravert et al., J. Label Compd. Radiopharm 2016, 59, 439-50; Bouvet et al., EJNMMMI Research, 2016, 6: 40). In einer Mischung aus 23,5 ml Trifluoressigsäure, 0,62 ml Triisopropylsilan und 0,62 ml Wasser wurden 2,48 g der Ausgangsverbindung XX gelöst und für 3 h bei Raumtemperatur gerührt. Im Anschluß wurde das Reaktionsgemisch unter Eisbadkühlung und starkem Rühren in 241 ml MTB-Ether getropft. Der ausgefallene weiße Feststoff wurde über eine Fritte abgesaugt und zweimal mit 100 ml MTB-Ether gewaschen. Es wurden 1,82 g (84 %) Präkursor der M 17 3 920 Neue Beschreibung RS.doc

Formel IVb (= 5-((*S*)-5-Carboxy-5-(3-((*S*)-1,3-dicarboxy-propyl)ureido)pentylcarbamoyl)-*N*,*N*,*N*-trimethylpyridin-2-aminium-2,2,2-trifluor-acetat) als weißer Feststoff isoliert.

### Beispiel 2

### Synthese eines Präkursors der Formel Vb

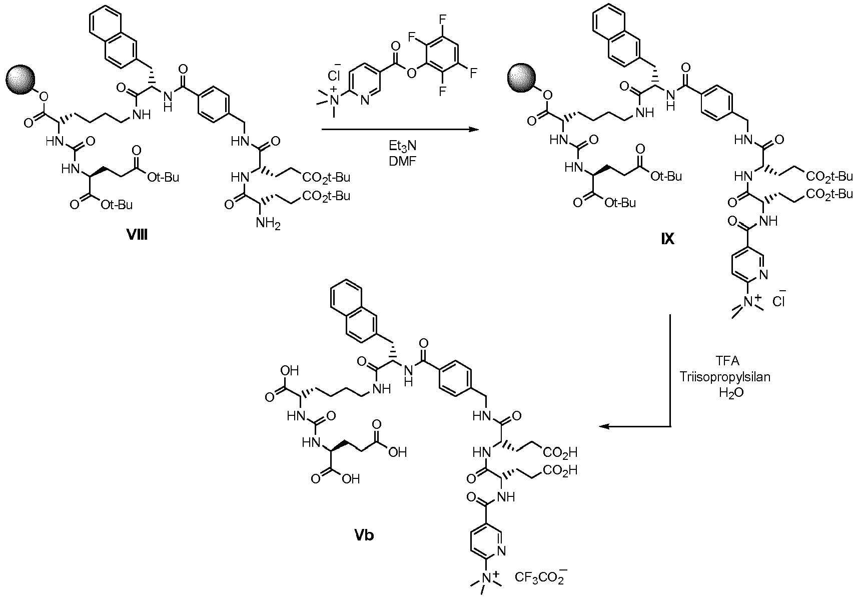

Die Synthese der Ausgangsverbindung VIII erfolgte analog wie in der Literatur beschrieben (Cardinale et al., J. Nucl. Med. 2016**,** accepted for publication; WO 2015/062370 A1). 0,2 mmol der Ausgangsverbindung VIII wurden in 3,5 ml Dimethylformamid für 30 min geschüttelt. Danach erfolgte die Zugabe von 109 mg *N*,*N*,*N-*Trimethyl-5-((2,3,5,6-tetrafluorphenoxy)-carbonyl)pyridin-2-aminiumchlorid (Olberg et al., J. Med. Chem. 2010, 53, 1732-1740) und 0,042 ml Triethylamin. Das Reaktionsgemisch wurde für 2 h geschüttelt, bevor das Harz filtriert und dreimal mit DMF und dreimal mit Dichlormethan gewaschen wurde. Zur Abspaltung und Entschützung wurde das Harz in einer Mischung aus 4 ml Trifluoressigsäure, 0,11 ml ml Triisopropylsilan und 0,11 ml Wasser für 90 min geschüttelt. Im Anschluss wurde das Gemisch filtiert, und das Filtrat wurde in 40 ml MTB-Ether getropft. Die Mischung wurde zentrifugiert, die überstehende Lösung abpipettiert und der Rückstand dreimal mit MTB-Ether gewaschen. Die Reinigung erfolgte mittels HPLC. Es wurden 172 mg (72%) Präkursor der Formel Vb (= 5-((*S*)-4-Carboxy-1-((*S*)-4-carboxy-1-(4-((*S*)-1-((*S*)-5-carboxy-5-(3-((*S*)-1,3-dicarboxy-propyl)-ureido)pentylamino)-3-(naphthalen-2-yl)-1-oxopropan-2-ylcarbamoyl)-benzylamino)-1-oxobutan-2-ylamino)-1-oxobutan-2-ylcarbamoyl)-*N*,*N*,*N*-trimethylpyridin-2-aminium-2,2,2-trifluoracetat) als weißer Feststoff isoliert.

### Beispiel 3

### Umsetzung eines Präkursors der Formel IVb in Gegenwart von Tetra-n-Butyl-Ammonium-Hydrogencarbonat zu [¹⁸F]-DCFPyL

Ein Reaktionsgemisch aus 7,5 mg Präkursor der Formel IVb in 1 ml DMF, 1 ml 0,075 M Tetra-*n*-Butyl-Ammonium-Hydrogencarbonat (TBA-HCO₃) und [¹⁸F]-FluoridAnionen wurde bei einem pH-Wert von etwa 8,5 für 14 min bei 75 °C umgesetzt. Es wurden 47,9 % [¹⁸F]-DCFPyL erhalten. Darüber hinaus konnten radioaktive Nebenverbindungen detektiert werden. Der Anteil an [¹⁸F]-Fluorid betrug 28,6 %.

### Beispiel 4

### Umsetzung eines Präkursors der Formel IVb in Gegenwart von Tetra-n-Butyl-Ammonium-Toluensulfonat zu [¹⁸F]-DCFPyL

Ein Reaktionsgemisch aus 7,5 mg Präkursor der Formel IVb in 1 ml DMF, 750 µl 0,075 M Tetra-n-Butyl-Ammonium-Toluensulfonat (TBA-Toluensulfonat) und [¹⁸F]-Fluorid-Anionen wurde bei einem pH-Wert von etwa 5,0 für 14 min bei 75 °C umgesetzt. Es wurden 37,4 % [¹⁸F]-DCFPyL und 30,9 % [¹⁸F]-Fluorid detektiert. Daneben wurden radioaktive Nebenverbindungen detektiert, deren Anteil ca. 30% betrug.

### Beispiel 5

### Umsetzung eines Präkursors der Formel IVb in Gegenwart von Tetra-n-Butyl-Ammonium-Phosphat zu [¹⁸F]-DCFPyL

Ein Reaktionsgemisch aus 2,5 mg Präkursor der Formel IVb in 1,5 ml DMF, 750 µl 0,075 M Tetra-n-Butyl-Ammonium-Phosphat (TBA-Phosphat) und [¹⁸F]-FluoridAnionen wurde bei einem pH-Wert von etwa 4,7 für 10 min bei 85 °C umgesetzt. Unter diesen Bedingungen wurde der Präkursor nahezu quantitativ in [¹⁸F]-DCFPyL überführt (97,0 %). Die Nebenverbindungen konnten unter 2% reduziert werden, restliches [¹⁸F]-Fluorid war nur noch in Spuren nachzuweisen.

### Beispiel 6

### Umsetzung eines Präkursors der Formel IVb in Gegenwart von Tetra-n-Butyl-Ammonium-Hydrogensulfat zu [¹⁸F]-DCFPyL

Ein Reaktionsgemisch aus 7,5 mg Präkursor der Formel IVb in 1 ml DMF, 750 µl 0,075 M Tetra-*n*-Butyl-Ammonium-Hydrogensulfat (TBA-Hydrogensulfat) und [¹⁸F]-Fluorid-Anionen wurde bei einem pH-Wert von etwa 1,7 für 14 min bei 75 °C umgesetzt. Es wurden 15,6 % [¹⁸F]-DCFPyL erhalten, der Anteil an [¹⁸F]-Fluorid betrug hingegen 73,4 %. Die Markierung mit [¹⁸F]-Fluorid-Anionen war somit vergleichsweise schlecht.

Die Beispiele 3 bis 6 zeigen, dass die Umsetzung von Präkursoren der Formel IVb mit [¹⁸F]-Fluorid-Anionen in Gegenwart von TBA als Aktivierungssalz in einem einstufigen Verfahren vergleichsweise hohe Ausbeuten der radiofluorierten Verbindung [¹⁸F]-DCFPyL ergibt. Beispiel 5 zeigt, dass im leicht sauren pH-Bereich mit TBA-Phosphat nur Spuren der Nebenverbindungen entstehen und eine extrem hohe Markierungsausbeute erzielt werden kann.

### Beispiel 7

### Umsetzung eines Präkursors der Formel Vb in Gegenwart von Tetra-n-Butyl-Ammonium-Hydrogencarbonat zu [¹⁸F]-PSMA-1007

Ein Reaktionsgemisch aus 10 mg Präkursor der Formel Vb in einem Gemisch aus 1 ml Acetonitril und 600 µl DMF, 750 µl 0,075 M TBA-Hydrogencarbonat und [¹⁸F]-Fluorid-Anionen wurde bei einem pH-Wert von ca. 7 für 10 min bei 120 °C inkubiert. Es wurden 59,3 % [¹⁸F]F-PSMA-1007 neben 37,9% freiem [¹⁸F]-Fluorid detektiert. In Spuren konnte ein radioaktives Nebenprodukt detektiert werden.

### Beispiel 8

### Umsetzung eines Präkursors der Formel Vb in Gegenwart von Tetra-n-Butyl-Ammonium-Hydrogencarbonat zu [¹⁸F]-PSMA-1007

Ein Reaktionsgemisch aus 2,5 mg Präkursor der Formel Vb in 1,5 ml DMF, 750 µl 0,075 M TBA-Hydrogencarbonat und [¹⁸F]-Fluorid-Anionen wurde bei einem pH-Wert von ca. 7 für 10 min bei 85 °C inkubiert. Es wurden 90,7 % [¹⁸F]-PSMA-1007 neben 8,2 % freiem [¹⁸F]-Fluorid detektiert. In Spuren konnte ein radioaktives Nebenprodukt detektiert werden.

### Beispiel 9

### Umsetzung eines Präkursors der Formel Vb in Gegenwart von Tetra-n-Butyl-Ammonium-phosphat zu [¹⁸F]-PSMA-1007

Ein Reaktionsgemisch aus 2,5 mg Präkursor der Formel Vb in 1,5 ml DMF, 750 µl 0,075 M TBA-phosphat und [¹⁸F]-Fluorid-Anionen wurde bei einem pH-Wert von ca. 4,7 für 10 min bei 85 °C inkubiert. Das gewünschte Produkt [¹⁸F]-PSMA-1007 wurde quantitativ gebildet (99,6%). Freies [¹⁸F]-Fluorid konnte nur in Spuren detektiert werden.

Die Beispiele 7 bis 9 zeigen, dass die Umsetzung von Präkursoren der Formel Vb mit [¹⁸F]-Fluorid-Anionen in Gegenwart von TBA als Aktivierungssalz in einem einstufigen Verfahren vergleichsweise hohe Ausbeuten der radiofluorierten Verbindung [¹⁸F]-PSMA-1007 ergibt. Beispiel 9 zeigt, dass im leicht sauren pH-Bereich mit TBA-Phosphat eine quantitative Markierungsausbeute erzielt werden kann.

### Beispiel 10

### Vollautomatisierte Umsetzung eines Präkursors der Formel Vb in Gegenwart von Tetra-n-Butyl-Ammonium-hydrogencarbonat zu [¹⁸F]-PSMA-1007 mit Hilfe eines Synthesemoduls GE TRACERlab^{(R)} MX_{FDG}

Mit lösungsmittelresistenten Hahnbänken wurde eine Kassette zur Synthese von [¹⁸F]-PSMA-1007 in Analogie zu einer FDG-Synthese-Kassette auf einem GE TRACERlab^{(R)} MX_{FDG} etabliert und eine Synthesesequenz entwickelt. Die Synthese verläuft im einzelnen nach folgenden Schritten ab: Aufkonzentrieren des [¹⁸F]-Fluorids auf einer QMA-Kartusche, Elution mit 0,750 ml TBA-Hydrogencarbonat und anschließender Trocknung bei 95 °C für 15 min, Radiomarkierung mit 3 mg Präkursor der Formel Vb in 2 ml DMF für 14 min bei 85 °C, SPE-Reinigung und Reformulierung. [¹⁸F]-PSMA-1007 konnte in radiochemischen Ausbeuten >40% erhalten werden. Die radiochemische Reinheit betrug >95%.

## Patentansprüche

1. Verfahren zur Herstellung einer radiofluorierten Verbindung, die einen aromatischen oder heteroaromatischen Ring, der [¹⁸F]-Fluor als ersten Substituenten trägt, eine Anbindungseinheit, die zur Anbindung an ein Peptid in der Lage ist, sowie eine Spacergruppe aufweist, die den aromatischen oder heteroaromatischen Ring mit der Anbindungseinheit verbindet, wobei der aromatische oder heteroaromatische Ring [¹⁸F]-Fluor als ersten Substituenten trägt, wobei die Anbindungseinheit zumindest einen zweiten Substituenten trägt, der aus der Gruppe ausgewählt ist, die aus -OH, -CONH und -COOH besteht, wobei die Anbindungseinheit mit der Spacergruppe über eine Bindung A¹ und die Spacergruppe über eine Bindung A² mit dem aromatischen oder heteroaromatischen Ring verbunden ist und wobei das Verfahren die Schritte umfasst:
(a) Bereitstellen eines Präkursors, der den aromatischen oder heteroaromatischen Ring, der einen Substituenten Y trägt, die Anbindungseinheit, die zur Anbindung an das Peptid in der Lage ist und die zumindest einen zweiten Substituenten, der aus der Gruppe ausgewählt ist, die aus -OH, - CONH und -COOH besteht, trägt, sowie die Spacergruppe aufweist, wobei der aromatische oder heteroaromatische Ring, der einen Substituenten Y trägt, die allgemeine Formel VId 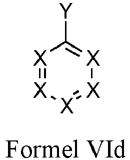 aufweist, wobei
- X jeweils C-R⁸ oder N ist, mit der Maßgabe, dass höchstens zwei der Einheiten X N und der Rest der Einheiten X C-R⁸ sind und R⁸ unabhängig voneinander jeweils die Bindung A² zum Spacer, Wasserstoff oder unsubstituiertes oder substituiertes C₁-C₆-Alkyl sind, mit der Maßgabe, dass exakt ein Rest R⁸ eine Bindung A² zur Spacergruppe ist und die verbleibenden R⁸ gleich oder verschieden voneinander sind und jeweils Wasserstoff oder unsubstituiertes oder substituiertes C₁-C₆-Alkyl darstellen; und
- der Substituent Y aus der Gruppe ausgewählt ist, die aus -N⁺(R¹R²R³), -NO₂, -Cl, -Br, -F oder -I besteht, und R¹, R² und R³ gleich oder verschieden voneinander sind und jeweils unsubstituiertes oder substituiertes C₁-C₆-Alkyl sind,
wobei sich der Präkursor von der radiofluorierten Verbindung nur dadurch unterscheidet, dass der Substituent Y durch [¹⁸F]-Fluor ersetzt ist; und
(b) Umsetzen des Präkursors mit einem [¹⁸F]-Fluorid-Anion in Gegenwart eines Aktivierungssalzes zu der radiofluorierten Verbindung in einer einstufigen Synthese, wobei der Substituent Y durch [¹⁸F]-Fluorid ersetzt wird und wobei das Aktivierungssalz ein Kation mit der allgemeinen Formel N⁺(R⁴R⁵R⁶R⁷) aufweist, wobei R⁴, R⁵, R⁶ und R⁷ gleich oder verschieden voneinander sind und jeweils unsubstituiertes oder substituiertes C₁-C₆-Alkyl sind.

2. Verfahren nach Anspruch 1, **dadurch gekennzeichnet, dass** R¹, R² und R³ jeweils Methyl sind.

3. Verfahren nach einem der vorstehenden Ansprüche, **dadurch gekennzeichnet, dass** R⁴, R⁵, R⁶ und R⁷ jeweils *n*-Butyl sind.

4. Verfahren nach einem der vorstehenden Ansprüche, **dadurch gekennzeichnet, dass** das Aktivierungssalz ein Anion aufweist, das aus der Gruppe ausgewählt ist, die Hydrogencarbonat, Hydrogensulfat, Oxalat, Phosphat und Toluensulfonat umfasst.

5. Verfahren nach einem der vorstehenden Ansprüche, **dadurch gekennzeichnet, dass** das Aktivierungssalz Tetra-*n*-Butyl-Ammonium-Hydrogencarbonat ist.

6. Verfahren nach einem der vorstehenden Ansprüche, **dadurch gekennzeichnet, dass** die Anbindungseinheit eine Anbindungseinheit der allgemeinen Formel I ist: 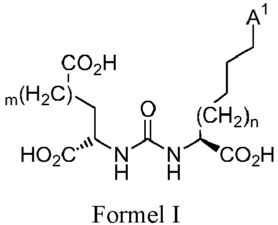 wobei A¹ die Bindung ist, über die die Anbindungseinheit mit der Spacergruppe verbunden ist, m und n gleich oder verschieden voneinander sind und jeweils eine Ganzzahl von 0 bis 10 sind.

7. Verfahren nach einem der vorstehenden Ansprüche, **dadurch gekennzeichnet, dass** die Spacergruppe eine Spacergruppe der allgemeinen Formel II oder der allgemeinen Formel III ist: 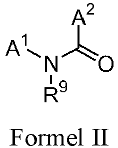 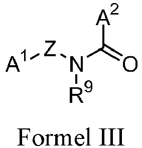 wobei A¹ die Bindung ist, über die die Spacergruppe mit der Anbindungseinheit verbunden ist, A² die Bindung ist, über die die Spacergruppe mit dem aromatischen oder heteroaromatischen Ring des Präkursors oder der radiofluorierten Verbindung verbunden ist, R⁹ Wasserstoff oder eine unsubstituierte oder substituierte C₁-C₆-Alkylgruppe ist, und Z ein unsubstituierter oder ein- oder mehrfach substituierter Kohlenwasserstoff ist.

8. Verfahren nach Anspruch 7, **dadurch gekennzeichnet, dass** Z eine Gruppe der Formel VII ist:

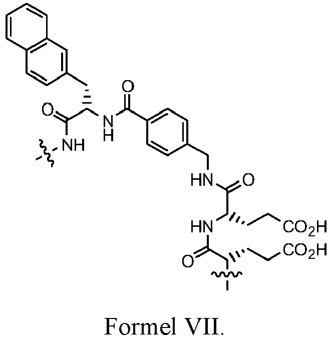

9. Verwendung eines Präkursors für die Herstellung einer radiofluorierten Verbindung in einer einstufigen Synthese, wobei der Präkursor einen aromatischen oder heteroaromatischen Ring, der einen Substituenten Y trägt, eine Anbindungseinheit, die zur Anbindung an ein Peptid in der Lage ist, sowie eine Spacergruppe aufweist, die den aromatischen oder heteroaromatischen Ring mit der Anbindungseinheit verbindet, wobei die Anbindungseinheit zumindest einen zweiten Substituenten trägt, der aus der Gruppe ausgewählt ist, die aus -OH, -CONH und -COOH besteht, wobei die Anbindungseinheit mit der Spacergruppe über eine Bindung A¹ und die Spacergruppe über eine Bindung A² mit dem aromatischen oder heteroaromatische Ring verbunden ist, wobei der aromatische oder heteroaromatische Ring, der einen Substituenten Y trägt, die allgemeine Formel VId 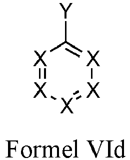 aufweist, wobei
- X jeweils C-R⁸ oder N ist, mit der Maßgabe, dass höchstens zwei der Einheiten X N und der Rest der Einheiten X C-R⁸ sind und R⁸ unabhängig voneinander jeweils die Bindung A² zum Spacer, Wasserstoff oder unsubstituiertes oder substituiertes C₁-C₆-Alkyl sind, mit der Maßgabe, dass exakt ein Rest R⁸ eine Bindung A² zur Spacergruppe ist und die verbleibenden R⁸ gleich oder verschieden voneinander sind und jeweils Wasserstoff oder unsubstituiertes oder substituiertes C₁-C₆-Alkyl darstellen; und
- der Substituent Y aus der Gruppe ausgewählt ist, die aus -N⁺(R¹R²R³), - NO₂, -Cl, -Br, -F oder -I besteht, und R¹, R² und R³ gleich oder verschieden voneinander sind und jeweils unsubstituiertes oder substituiertes C₁-C₆-Alkyl sind,
wobei sich der Präkursor von der radiofluorierten Verbindung nur dadurch unterscheidet, dass der Substituent Y durch [¹⁸F]-Fluor ersetzt ist.

10. Verwendung eines Präkursors nach Anspruch 9, **dadurch gekennzeichnet, dass** die Anbindungseinheit eine Anbindungseinheit der allgemeinen Formel I ist: 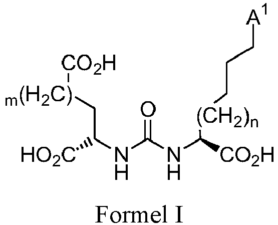 wobei A¹ die Bindung ist, über die die Anbindungseinheit mit der Spacergruppe verbunden ist, und m und n gleich oder verschieden voneinander sind und jeweils eine Ganzzahl von 0 bis 10 sind.

11. Verwendung eines Präkursors nach Anspruch 9 oder Anspruch 10, **dadurch gekennzeichnet, dass** die Spacergruppe eine Spacergruppe der allgemeinen Formel II oder der allgemeinen Formel III ist: 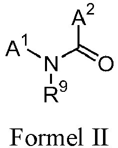 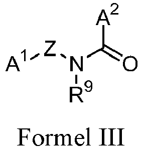 wobei A¹ die Bindung ist, über die die Spacergruppe mit der Anbindungseinheit verbunden ist, A² die Bindung ist, über die die Spacergruppe mit dem aromatischen oder heteroaromatischen Ring des Präkursors oder der radiofluorierten Verbindung verbunden ist, R⁹ Wasserstoff oder eine unsubstituierte oder substituierte C₁-C₆-Alkylgruppe ist, und Z ein unsubstituierter oder ein- oder mehrfach substituierter Kohlenwasserstoff ist.

12. Verbindung, die einen aromatischen oder heteroaromatischen Ring, der einen Substituenten Y trägt, eine Anbindungseinheit, die zur Anbindung an ein Peptid in der Lage ist, sowie eine Spacergruppe aufweist, die den aromatischen oder heteroaromatischen Ring mit der Anbindungseinheit verbindet, wobei die Anbindungseinheit zumindest einen zweiten Substituenten trägt, der aus der Gruppe ausgewählt ist, die aus -OH, -CONH und -COOH besteht, wobei die Anbindungseinheit mit der Spacergruppe über eine Bindung A¹ und die Spacergruppe über eine Bindung A² mit dem aromatischen oder heteroaromatische Ring verbunden ist, wobei der aromatische oder heteroaromatische Ring, der einen Substituenten Y trägt, die allgemeine Formel VId 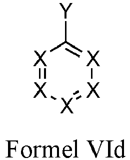 aufweist, wobei
- X jeweils C-R⁸ oder N ist, mit der Maßgabe, dass höchstens zwei der Einheiten X N und der Rest der Einheiten X C-R⁸ sind und R⁸ unabhängig voneinander jeweils die Bindung A² zum Spacer, Wasserstoff oder unsubstituiertes oder substituiertes C₁-C₆-Alkyl sind, mit der Maßgabe, dass exakt ein Rest R⁸ eine Bindung A² zur Spacergruppe ist und die verbleibenden R⁸ gleich oder verschieden voneinander sind und jeweils Wasserstoff oder unsubstituiertes oder substituiertes C₁-C₆-Alkyl darstellen; und
- der Substituent Y aus der Gruppe ausgewählt ist, die aus -N+(R¹R²R³), -NO₂, -Cl, -Br oder -I besteht, und R¹, R² und R³ gleich oder verschieden voneinander sind und jeweils unsubstituiertes oder substituiertes C₁-C₆-Alkyl sind,
- die Spacergruppe eine Spacergruppe der allgemeinen Formel II oder der allgemeinen Formel III ist: 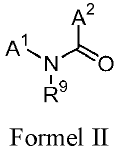 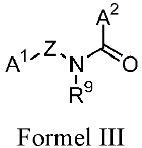 wobei A¹ die Bindung ist, über die die Spacergruppe mit der Anbindungseinheit verbunden ist, A² die Bindung ist, über die die Spacergruppe mit dem aromatischen oder heteroaromatischen Ring des Präkursors oder der radiofluorierten Verbindung verbunden ist, R⁹ Wasserstoff oder eine unsubstituierte oder substituierte C₁-C₆-Alkylgruppe ist, und Z ein unsubstituierter oder ein- oder mehrfach substituierter Kohlenwasserstoff ist, **dadurch gekennzeichnet, dass** Z eine Gruppe der Formel VII ist:

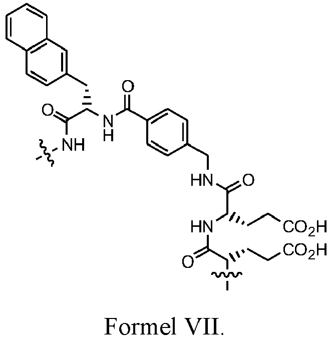

13. Verbindung der Formel IVa: 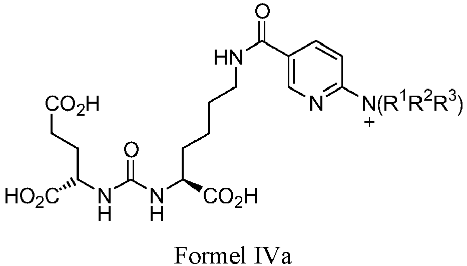 wobei R¹, R² und R³ gleich oder verschieden voneinander sind und jeweils unsubstituiertes oder substituiertes C₁-C₆-Alkyl sind.

14. Verbindung der Formel Va: 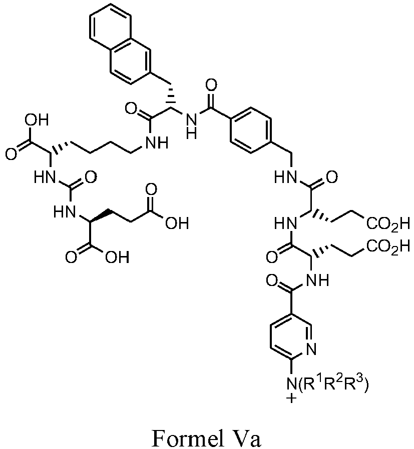 wobei R¹, R² und R³ gleich oder verschieden voneinander sind und jeweils unsubstituiertes oder substituiertes C₁-C₆-Alkyl sind.

15. Verwendung eines Präkursors nach einem der Ansprüche 12 bis 14 zur Herstellung einer radiofluorierten Verbindung in einer einstufigen Synthese, die einen aromatischen oder heteroaromatischen Ring, der [¹⁸F]-Fluor als ersten Substituenten trägt, eine Anbindungseinheit, die zur Anbindung an ein Peptid in der Lage ist, sowie eine Spacergruppe aufweist, die den aromatischen oder heteroaromatischen Ring mit der Anbindungseinheit verbindet, wobei die Anbindungseinheit zumindest einen zweiten Substituenten trägt, der aus der Gruppe ausgewählt ist, die aus -OH, -CONH und -COOH besteht, wobei die Anbindungseinheit mit der Spacergruppe über eine Bindung A¹ und die Spacergruppe über eine Bindung A² mit dem aromatischen oder heteroaromatischen Ring verbunden ist, wobei sich der Präkursor von der radiofluorierten Verbindung nur dadurch unterscheidet, dass der Substituent Y durch [¹⁸F]-Fluor ersetzt ist.

## Claims

1. A method for producing a radiofluorinated compound which has an aromatic or heteroaromatic ring which carries [¹⁸F] fluorine as a first substituent, a bonding unit which can bind to a peptide, as well as a spacer group which connects the aromatic or heteroaromatic ring to the bonding unit, wherein the aromatic or heteroaromatic ring carries [¹⁸F] fluorine as the first substituent, wherein the bonding unit carries at least one second substituent selected from the group consisting of -OH, -CONH, and -COOH, wherein the bonding unit is connected to the spacer group via a bond A¹ and the spacer group is connected to the aromatic or heteroaromatic ring via a bond A² and wherein the method comprises the steps of:
(a) providing a precursor which has the aromatic or heteroaromatic ring which carries a substituent Y, the bonding unit which can bind to the peptide and which carries at least one second substituent selected from the group consisting of -OH, -CONH, and -COOH, as well as the spacer group, wherein the aromatic or heteroaromatic ring, which carries a substituent Y, is of general formula VId 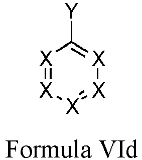 wherein
- X each is C-R⁸ or N, with the provision that at most two of the moieties X are N and the remaining of the moieties X are C-R⁸ and R⁸ each independently is the bond A² to the spacer, hydrogen, or unsubstituted or substituted C₁-C₆ alkyl, with the provision that exactly one residue R⁸ is a bond A² to the spacer group and the remaining ones of R⁸ are the same or different from each other and each represent hydrogen or unsubstituted or substituted C₁-C₆ alkyl; and
- the substituent Y is selected from the group consisting of -N+(R¹R²R³), -NO₂, -Cl, -Br, -F, or -I, and R¹, R² and R³ are the same or different from each other and each are unsubstituted or substituted C₁-C₆ alkyl,
wherein the precursor only differs from der radiofluorinated compound in that the substituent Y is replaced by [¹⁸F] fluorine; and
(b) reacting the precursor with a [¹⁸F] fluoride anion in the presence of an activation salt to the radiofluorinated compound in a one-stage synthesis, wherein the substituent Y is replaced by [¹⁸F] fluoride and wherein the activation salt has a cation of general formula N⁺(R⁴R⁵R⁶R⁷), wherein R⁴, R⁵, R⁶, and R⁷ are the same or different from each other and each are unsubstituted or substituted C₁-C₆ alkyl.

2. The method according to claim 1, **characterized in that** R¹, R², and R³ each are methyl.

3. The method according to any of the preceding claims, **characterized in that** R⁴, R⁵, R⁶, and R⁷ each are *n*-butyl.

4. The method according to any of the preceding claims, **characterized in that** the activation salt has an anion selected from the group comprising hydrogen carbonate, hydrogen sulphate, oxalate, phosphate, and toluenesulphonate.

5. The method according to any of the preceding claims, **characterized in that** the activation salt is tetra-*n*-butyl-ammonium hydrogen carbonate.

6. The method according to any of the preceding claims, **characterized in that** the bonding unit is a bonding unit of general formula I: 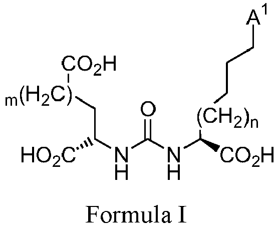 wherein A¹ is the bond via which the bonding unit is connected to the spacer group, m and n are the same or different from each other and each are an integer of from 0 to 10.

7. The method according to any of the preceding claims, **characterized in that** the spacer group is a spacer group of general formula II or general formula III: 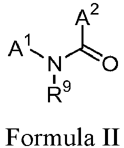 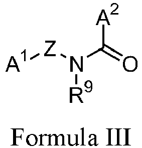 wherein A¹ is the bond via which the spacer group is connected to the bonding unit, A² is the bond via which the spacer group is connected to the aromatic or heteroaromatic ring of the precursor or to the radiofluorinated compound, R⁹ is hydrogen or an unsubstituted or substituted C₁-C₆ alkyl group, and Z is an unsubstituted or mono- or poly-substituted hydrocarbon.

8. The method according to claim 7, **characterized in that** Z is a group of formula VII:

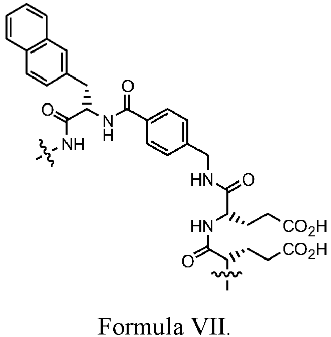

9. Use of a precursor for producing a radiofluorinated compound in a one-stage synthesis, wherein the precursor has an aromatic or heteroaromatic ring which carries a substituent Y, a bonding unit which can bind to a peptide, as well as a spacer group which connects the aromatic or heteroaromatic ring to the bonding unit, wherein the bonding unit carries at least one second substituent selected from the group consisting of -OH, -CONH, and -COOH, wherein the bonding unit is connected to the spacer group via a bond A¹ and the spacer group is connected to the aromatic or heteroaromatic ring via a bond A², wherein the aromatic or heteroaromatic ring, which carries a substituent Y, is of general formula VId 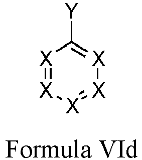 wherein
- X each is C-R⁸ or N, with the provision that at most two of the moieties X are N and the remaining of the moieties X are C-R⁸ and R⁸ each independently is the bond A² to the spacer, hydrogen, or unsubstituted or substituted C₁-C₆ alkyl, with the provision that exactly one residue R⁸ is a bond A² to the spacer group and the remaining ones of R⁸ are the same or different from each other and each represent hydrogen or unsubstituted or substituted C₁-C₆ alkyl; and
- the substituent Y is selected from the group consisting of -N+(R¹R²R³), -NO₂, -Cl, -Br, -F, or -I, and R¹, R² and R³ are the same or different from each other and each are unsubstituted or substituted C₁-C₆ alkyl,
wherein the precursor only differs from der radiofluorinated compound in that the substituent Y is replaced by [¹⁸F] fluorine.

10. Use of a precursor according to claim 9, **characterized in that** the bonding unit is a bonding unit of general formula I: 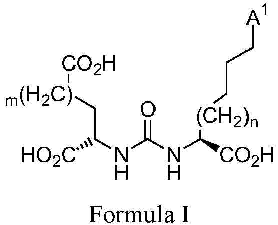 wherein A¹ is the bond via which the bonding unit is connected to the spacer group, and m and n are the same or different from each other and each are an integer of from 0 to 10.

11. Use of a precursor according to claim 9 or claim 10, **characterized in that** the spacer group is a spacer group of general formula II or of general formula III: 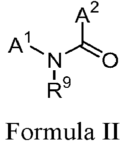 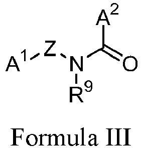 wherein A¹ is the bond via which the spacer group is connected to the bonding unit, A² is the bond via which the spacer group is connected to the aromatic or heteroaromatic ring of the precursor or to the radiofluorinated compound, R⁹ is hydrogen or an unsubstituted or substituted C₁-C₆ alkyl group, and Z is an unsubstituted or mono- or poly-substituted hydrocarbon.

12. A compound having an aromatic or heteroaromatic ring which carries a substituent Y, a bonding unit which can bind to a peptide, as well as a spacer group which connects the aromatic or heteroaromatic ring to the bonding unit, wherein the bonding unit carries at least one second substituent selected from the group consisting of -OH, -CONH, and -COOH, wherein the bonding unit is connected to the spacer group via a bond A¹ and the spacer group is connected to the aromatic or heteroaromatic ring via a bond A², wherein the aromatic or heteroaromatic ring, which carries a substituent Y, is of general formula VId 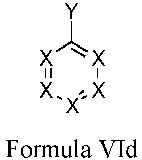 wherein
- X each is C-R⁸ or N, with the provision that at most two of the moieties X are N and the remaining of the moieties X are C-R⁸ and R⁸ each independently is the bond A² to the spacer, hydrogen, or unsubstituted or substituted C₁-C₆ alkyl, with the provision that exactly one residue R⁸ is a bond A² to the spacer group and the remaining ones of R⁸ are the same or different from each other and each represent hydrogen or unsubstituted or substituted C₁-C₆ alkyl; and
- the substituent Y is selected from the group consisting of -N+(R¹R²R³), -NO₂, -Cl, -Br, or -I, and R¹, R² and R³ are the same or different from each other and each are unsubstituted or substituted C₁-C₆ alkyl,
- the spacer group is a spacer group of general formula II or of general formula III: 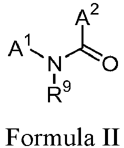 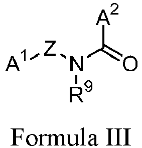 wherein A¹ is the bond via which the spacer group is connected to the bonding unit, A² is the bond via which the spacer group is connected to the aromatic or heteroaromatic ring of the precursor or to the radiofluorinated compound, R⁹ is hydrogen or an unsubstituted or substituted C₁-C₆ alkyl group, and Z is an unsubstituted or mono- or poly-substituted hydrocarbon,
**characterized in that** Z is a group of formula VII:

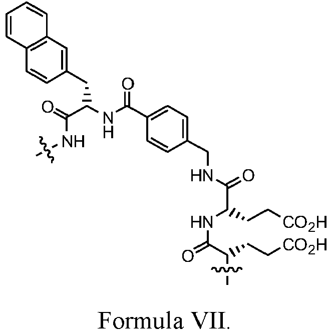

13. A compound of formula IVa: 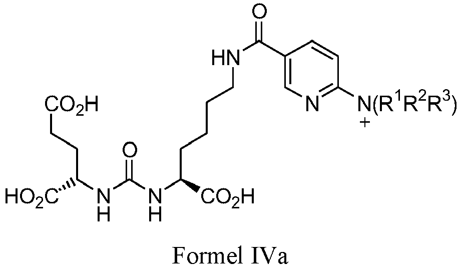 wherein R¹, R², and R³ are the same or different from each other and each are unsubstituted or substituted C₁-C₆ alkyl.

14. A compound of formula Va: 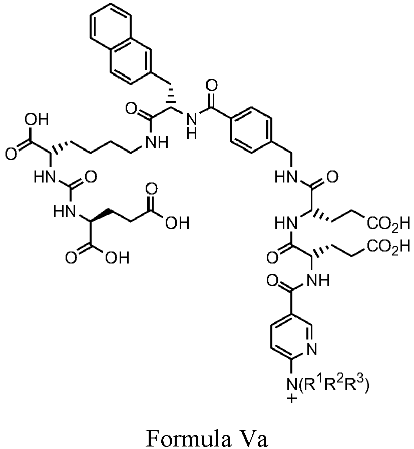 wherein R¹, R², and R³ are the same or different from each other and each are unsubstituted or substituted C₁-C₆ alkyl.

15. Use of a precursor according to any of claims 12 to 14 for producing a radiofluorinated compound in a one-stage synthesis which has an aromatic or heteroaromatic ring which carries [¹⁸F] fluorine as a first substituent, a bonding unit which can bind to a peptide, as well as a spacer group which connects the aromatic or heteroaromatic ring to the bonding unit, wherein the bonding unit carries at least one second substituent selected from the group consisting of -OH, -CONH, and -COOH, wherein the bonding unit is connected to the spacer group via a bond A¹ and the spacer group is connected to the aromatic or heteroaromatic ring via a bond A², wherein the precursor only differs from the radiofluorinated compound in that the substituent Y is replaced by [¹⁸F] fluorine.

## Revendications

1. Procédé pour la préparation d'un composé radiofluoré comprenant un cycle aromatique ou hétéroaromatique portant du fluor [¹⁸F] en tant que premier substituant, une unité de liaison en mesure de se lier à un peptide, et un groupe espaceur reliant le cycle aromatique ou hétéroaromatique à l'unité de liaison, dans lequel le cycle aromatique ou hétéroaromatique porte du fluor [¹⁸F] comme premier substituant, ladite unité de liaison portant au moins un second substituant choisi dans le groupe composé de -OH, -CONH et -COOH, ladite unité de liaison étant liée audit groupe espaceur par une liaison A¹ et ledit groupe espaceur étant lié audit cycle aromatique ou hétéroaromatique par une liaison A², ledit procédé comprenant les étapes consistant à :
(a) mise à disposition d'un précurseur comprenant le cycle aromatique ou hétéroaromatique portant un substituant Y, l'unité de liaison en mesure de se lier au peptide et portant au moins un second substituant choisi dans le groupe composé de -OH, -CONH et -COOH, et le groupe espaceur, dans lequel le cycle aromatique ou hétéroaromatique portant un substituant Y, présentant la formule générale VId, 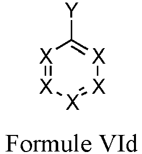
- X étant à chaque fois C-R⁸ ou N, à la condition qu'au maximum deux des unités X N et les autres unités soient C-R⁸ et R⁸ indépendamment les unes des autres soit à chaque fois la liaison A² avec l'espaceur, l'hydrogène ou un alkyle C₁-C₆ non substitué ou substitué, à condition qu'exactement un R⁸ restant soit une liaison A² avec le groupe espaceur et que les autres R⁸ soient identiques ou différents les uns des autres et représentent à chaque fois l'hydrogène ou un alkyle en C₁-C₆ non substitué ou substitué ; et
- le substituant Y étant sélectionné dans le groupe composé de -N+(R¹R²R³), -NO₂, -Cl, -Br, -F ou -I, et R¹, R² et R³ sont identiques ou différents les uns des autres et désignent respectivement alkyle C₁-C₆ non substitué ou substitué ,
le précurseur étant différent du composé radiofluoré uniquement en ce que le substituant Y est remplacé par du fluor [¹⁸F] ; et
(b) la réaction du précurseur avec un anion fluorure [¹⁸F] en présence d'un sel d'activation pour former le composé radiofluoré dans une synthèse en une étape, le substituant Y étant remplacé par du fluorure [¹⁸F] et le sel d'activation comprenant un cation avec la formule générale N⁺(R⁴R⁵R⁶R⁷), R⁴, R⁵, R⁶ et R⁷ étant identiques ou différents les uns des autres et étant chacun un alkyle C₁-C₆ non substitué ou substitué.

2. Procédé selon la revendication 1, **caractérisé en ce que** R¹, R² et R³ sont chacun un groupe méthyle.

3. Procédé selon l'une des revendications précédentes, **caractérisé en ce que** R⁴, R⁵, R⁶ et R⁷ sont chacun un *n*-butyle.

4. Procédé selon l'une des revendications précédentes, **caractérisé en ce que** le sel activateur comprend un anion choisi dans le groupe constitué par l'hydrogénocarbonate, l'hydrogénosulfate, l'oxalate, le phosphate et le toluènesulfonate.

5. Procédé selon l'une des revendications précédentes, **caractérisé en ce que** le sel activateur est de l'hydrogénocarbonate de tétra-*n*-butyle ammonium.

6. Procédé selon l'une des revendications précédentes, **caractérisé en ce que** l'unité de liaison est une unité de liaison de formule générale I : 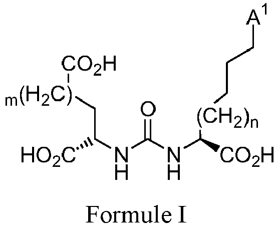 A¹ étant la liaison via laquelle l'unité de liaison est liée au groupe espaceur, m et n étant identiques ou différents l'un de l'autre et étant chacun un nombre entier de 0 à 10.

7. Procédé selon l'une des revendications précédentes, **caractérisé en ce que** le groupe espaceur est un groupe espaceur de la formule générale II ou de la formule générale III : 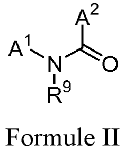 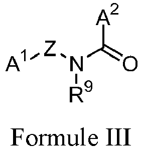 A¹ étant la liaison via laquelle le groupe espaceur et l'unité de liaison son liés, A² étant la liaison via laquelle le groupe espaceur est lié au cycle aromatique ou hétéroaromatique du précurseur ou du composé radiofluoré, R⁹ étant de l'hydrogène ou un groupe alkyle C₁-C₆ non substitué ou substitué, et Z un hydrocarbure non substitué ou mono- ou polysubstitué.

8. Procédé selon la revendication 7, **caractérisé en ce que** Z est un groupe de la formule VII :

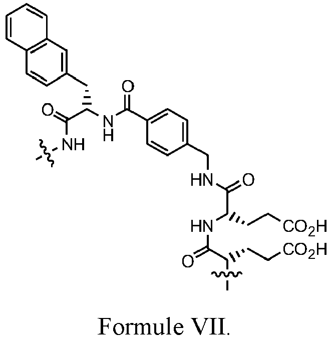

9. Utilisation d'un précurseur pour la préparation d'un composé radiofluoré dans une synthèse en une étape, ledit précurseur comprenant un cycle aromatique ou hétéroaromatique portant un substituant Y, une unité de liaison en mesure de se lier à un peptide, et un groupe espaceur reliant le cycle aromatique ou hétéroaromatique à l'unité de liaison, ladite unité de liaison portant au moins un second substituant choisi dans le groupe composé de -OH, -CONH et -COOH, ladite unité de liaison étant liée audit groupe espaceur par une liaison A¹ et ledit groupe espaceur étant lié audit cycle aromatique ou hétéroaromatique par une liaison A², dans lequel ledit cycle aromatique ou hétéroaromatique portant un substituant Y présentant la formule générale VId 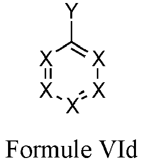
- X étant à chaque fois C-R⁸ ou N, à la condition qu'au maximum deux des unités X N et les autres unités soient C-R⁸ et R⁸ indépendamment les unes des autres soit à chaque fois la liaison A² avec l'espaceur, l'hydrogène ou un alkyle C₁-C₆ non substitué ou substitué, à condition qu'exactement un R⁸ restant soit une liaison A² avec le groupe espaceur et que les autres R⁸ soient identiques ou différents les uns des autres et représentent à chaque fois l'hydrogène ou un alkyle en C₁-C₆ non substitué ou substitué ; et
- le substituant Y étant sélectionné dans le groupe composé de -N⁺(R¹R²R³), -NO₂, -Cl, -Br, -F ou -I, et R¹, R² et R³ sont identiques ou différents les uns des autres et désignent respectivement alkyle C₁-C₆ non substitué ou substitué,
le précurseur étant différent du composé radiofluoré uniquement en ce que le substituant Y est remplacé par du fluor [¹⁸F].

10. Utilisation d'un précurseur selon la revendication 9, **caractérisée en ce que** l'unité de liaison est une unité de liaison de formule générale I : 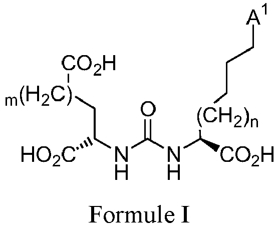 A¹ étant la liaison via laquelle l'unité de liaison est liée au groupe espaceur, et m et n étant identiques ou différents l'un de l'autre et étant chacun un nombre entier de 0 à 10.

11. Utilisation d'un précurseur selon la revendication 9 ou la revendication 10, **caractérisée en ce que** le groupe espaceur est un groupe espaceur de la formule générale II ou de la formule générale III : 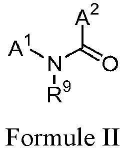 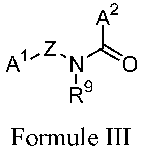 A¹ étant la liaison via laquelle le groupe espaceur et l'unité de liaison son liés, A² étant la liaison via laquelle le groupe espaceur est lié au cycle aromatique ou hétéroaromatique du précurseur ou du composé radiofluoré, R⁹ étant de l'hydrogène ou un groupe alkyle C₁-C₆ non substitué ou substitué, et Z un hydrocarbure non substitué ou mono- ou polysubstitué.

12. Composé comprenant un cycle aromatique ou hétéroaromatique portant un substituant Y, une unité de liaison en mesure de se lier à un peptide, et un groupe espaceur reliant le cycle aromatique ou hétéroaromatique à l'unité de liaison, ladite unité de liaison portant au moins un second substituant choisi dans le groupe composé de -OH, -CONH et -COOH, ladite unité de liaison étant liée audit groupe espaceur par une liaison A¹ et ledit groupe espaceur étant lié audit cycle aromatique ou hétéroaromatique par une liaison A², dans lequel ledit cycle aromatique ou hétéroaromatique portant un substituant Y présentant la formule générale VId 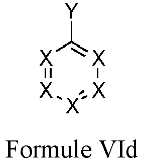
- X étant à chaque fois C-R⁸ ou N, à la condition qu'au maximum deux des unités X N et les autres unités soient C-R⁸ et R⁸ indépendamment les unes des autres soit à chaque fois la liaison A² avec l'espaceur, l'hydrogène ou un alkyle C₁-C₆ non substitué ou substitué, à condition qu'exactement un R⁸ restant soit une liaison A² avec le groupe espaceur et que les autres R⁸ soient identiques ou différents les uns des autres et représentent à chaque fois l'hydrogène ou un alkyle en C₁-C₆ non substitué ou substitué ; et
- le substituant Y étant sélectionné dans le groupe composé de -N⁺(R¹R²R³), -NO₂, -Cl, -Br ou -I, et R¹, R² et R³ étant identiques ou différents les uns des autres et désignent respectivement alkyle C₁-C₆ non substitué ou substitué,
- le groupe espaceur étant un groupe espaceur de la formule générale II ou de la formule générale III : 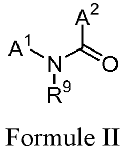 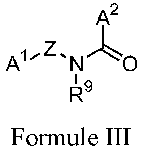 A¹ étant la liaison via laquelle le groupe espaceur et l'unité de liaison son liés, A² étant la liaison via laquelle le groupe espaceur est lié au cycle aromatique ou hétéroaromatique du précurseur ou du composé radiofluoré, R⁹ étant de l'hydrogène ou un groupe alkyle C₁-C₆ non substitué ou substitué, et Z un hydrocarbure non substitué ou mono- ou polysubstitué,
**caractérisé en ce que** Z est un groupe de la formule VII :

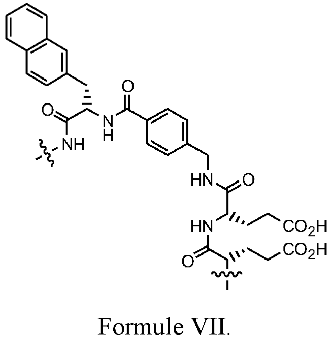

13. Composé de la formule IVa : 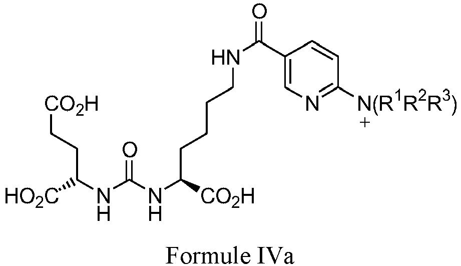 R¹, R² et R³ étant identiques ou différents les uns des autres et étant chacun un alkyle C₁-C₆ non substitué ou substitué.

14. Composé de la formule Va : 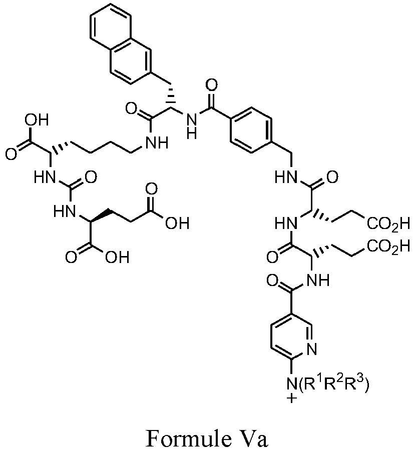 R¹, R² et R³ étant identiques ou différents les uns des autres et étant chacun un alkyle C₁-C₆ non substitué ou substitué.

15. Utilisation d'un précurseur selon l'une des revendications 12 à 14 pour la préparation d'un composé radiofluoré dans une synthèse en une étape comprenant un cycle aromatique ou hétéroaromatique portant du fluor [¹⁸F] comme premier substituant, une unité de liaison en mesure de se lier à un peptide, et un groupe espaceur reliant le cycle aromatique ou hétéroaromatique à l'unité de liaison, ladite unité de liaison portant au moins un second substituant choisi dans le groupe composé de -OH, -CONH et -COOH, ladite unité de liaison étant liée audit groupe espaceur par une liaison A¹ et ledit groupe espaceur étant lié audit cycle aromatique ou hétéroaromatique par une liaison A², ledit précurseur différant dudit composé radiofluoré uniquement en ce que le substituant Y est remplacé par du fluor [¹⁸F].
